# EUROPEAN PATENT SPECIFICATION

(11) **EP 1 951 653 B1**
(45) Date of publication and mention of the grant of the patent: **20.10.2010**
(21) Application number: 06829984.1
(22) Date of filing: 14.11.2006
(51) Int. Cl.: C07C 203/04, C07D 271/08, A61K 31/60, A61P 7/02, A61P 25/00, A61P 29/00, A61P 35/00

(54) **SALICYLIC ACID DERIVATIVES**
SALICYLSÄUREDERIVATE
DERIVES D ACIDE SALICYLIQUE

(30) Priority: 23.11.2005 US 738978 P
(43) Date of publication of application: 06.08.2008
(73) Proprietor: NicOx S.A., 06560 Sophia Antipolis - Valbonne (FR)
(72) Inventor: GASCO, Alberto, I-10024 Moncalieri (Torino) (IT); FRUTTERO, Roberta, I-12038 Savigliano (Cuneo) (IT); LAZZARATO, Loretta, I-10098 Rivoli (Torino) (IT); DONNOLA, Monica, I-10141 Torino (IT)
(74) Representative: Barchielli, Giovanna
(86) International application number: PCT/EP2006/068417
(87) International publication number: WO 2007/060112

(56) References cited:
- WO-A-00/51988
- WO-A-96/34848
- WO-A-97/16405
- WO-A-02/053188
- WO-A-02/100400
- WO-A-2004/000273
- WO-A-2004/000300
- WO-A-2005/030224
- WO-A2-02/11706
- ENDRES S ET AL: "NO-Donors, part 3: nitrooxyacylated thiosalicylates and salicylates - synthesis and biological activities" EUROPEAN JOURNAL OF MEDICINAL CHEMISTRY, EDITIONS SCIENTIFIQUE ELSEVIER, PARIS, FR, vol. 34, no. 11, November 1999 (1999-11), pages 895-901, XP004330429 ISSN: 0223-5234 cited in the application
- GOODMAN & GILMAN: "The Pharmacological Basis of Therapeutics, Tenth Edition" MCGRAW-HILL * page 690 - page 692 *

## Description

The present invention refers to O-acyl salicylic acid derivatives bearing a NO donor moiety, a process for their preparation and pharmaceutical compositions containing them.

WO 95/30641 discloses derivatives of acetyl salicylic acid wherein a moiety bearing a nitrooxy group is linked to the carboxylic group through an ester bond. These compounds have anti-inflammatory, analgesic and anti-thrombotic activity with lower gastrointestinal toxicity in comparison with acetyl salicilyc acid.

Endres et al., Eur.J.Med.Chem. 34 (1999),895-901, discloses o-acyl salicylic acid esters wherein the phenol group of salicylic acid is linked through an ester bond to an alkyl chain bearing a ONO₂ group. The study shows the ability of these compounds to release NO but no particular pharmacological property is reported.

WO 2004/000273 discloses pharmaceutical composition of liquid drugs, among which derivatives of acetyl salicylic acid wherein the moiety bearing a nitrooxy group is linked to the carboxylic group of acetyl salicylic acid through an ester bond. No particular pharmacological activity is described.

WO 2004/000300 discloses nitrooxy derivatives of NSAIDs able to selectively inhibit COX-2. Among others, derivatives of acetyl salicylic acid wherein a moiety bearing a nitrooxy group is linked to the carboxylic group through an ester bond are described.

WO 02/100400 discloses the use of derivatives of acetyl salicylic acid or salicyl acid wherein the carboxylic group is esterified with a moiety bringing a nitrooxy group for treating vasculopathies.

WO 02/053188 describes pharmaceutical composition comprising NO-releasing derivatives of drugs and cyclodextrins. Derivatives of acetyl salicylic acid or salicylic acid wherein the carboxylic group is esterified with a moiety bringing a nitrooxy group are among the NO-releasing derivatives.

WO 00/51988 describes derivatives of acetyl salicylic acid or salicyl acid wherein the carboxylic group is esterified with a moiety bringing a nitrooxy group. The moiety bringing the nitrooxy group contain an heterocyclic ring. The compounds are able to inhibit aorta and corpus cavernosum smooth muscle contraction and cell proliferation.

WO 02/11706 discloses the use of nitrooxy derivatives of drugs for the treatment of sex dysfunctions. In the general formula the acetyl salicylic acid or salicyl acid derivatives wherein the carboxylic group is esterified with a moiety bringing a nitrooxy group, are comprised.

WO 2005/030224 discloses the use of nitrooxy derivatives of NSAIDs for the treatment of viral diseases. In the general formula, the acetyl salicylic acid or salicyl acid derivatives wherein the carboxylic group is esterified with a moiety bringing a nitrooxy group, are comprised.

WO 96/34848 relates to nitrooxy derivatives of acetyl salicylic acid. The nitrooxy bearing moiety is linked to the carboxylic group of the acetyl salicylic acid through an ester bond. The compounds have at least the same activity as acetyl salicylic acid.

WO 97/16405 describes nitrooxy derivatives of acetyl salicylic acid, wherein the nitrooxy bearing moiety contains an aromatic ring and it is linked to the carboxylic group of the acetyl salicylic acid through an ester bond. The compounds have an anti-inflammatory and anti-thrombotic activity.

The present invention relates to novel O-acyl salicylic acid derivatives bearing a NO donor moiety. They have anti-inflammatory, analgesic, antipyretic, antithrombotic activities and vasodilating, platelet-antiaggregatory properties together with a reduced risk of gastric lesions and bleeding.

The compounds of the invention can be used for preventing and treating thrombotic cardiovascular events caused by platelet aggregation, thrombosis, and subsequent ischemic clinical events, including thrombotic or thromboembolic stroke, myocardial ischemia, myocardial infarction, angina pectoris, transient ischemic attack, reversible ischemic neurologic deficits, and any similar thrombotic event in any vascular bed (splanchnic, renal, aortic, peripheral, etc.).

The compounds of the invention are useful for the relief of pain, fever and inflammation of a variety of conditions including rheumatic fever, symptoms associated with influenza or other viral infections, common cold, low back and neck pain, dysmenorrhea, headhache, toothache, sprains and strains, myositis, neuralgia, synovitis, arthritis, including rheumatoid arthritis degenerative joint diseases (osteoarthritis), gout and ankylosing spondylitis, bursitis, burns, injuries, following surgical and dental procedures.

The compounds of the invention can be used alone or in combination with NSAIDs, such as those described in Goodman and Gilman's, The Pharmacological Basis of Therapeutics, Tenth Edition, p. 687-716.

The compounds of the present invention are useful in the prevention and treatment of cancer diseases in particular those affecting gastrointestinal and urogenital apparatus, such as colon cancer, bladder cancer and prostate cancer.

Object of the present invention are compounds of general formula (I) and pharmaceutically acceptable salts or stereoisomers thereof: 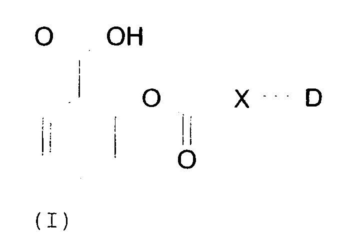 wherein:
D is -ONO₂ or 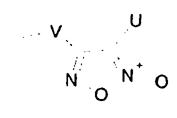 wherein V is -CH₂-, -O-, -S- or -NH-; U is C₁-C₁₀ alkyl, optionally substituted with -OH or -NH₂, aryl, C₁-C₁₀ alkoxy, aryloxy, C₁-C₁₀ thioalkyl, thioaryl, halogen, di-C₁-C₁₀(alkylamino), diarylamino; arylC₁-C₁₀ (alkylamino), C₁-C₁₀(alkylsulphoxy), arylsulphoxy, C₁-C₁₀ (alkylsulphone), arylsulphone, -CN, -NO₂, -NHCOR₀, -COR₀, -COOR₀, -CON(R₀)(R₁), wherein R₀ and R₁ are the same or different, and are H, alkyl or aryl;
X is a bivalent radical having the following meanings:
   a) straight or branched C₁-C₂₀ alkylene, optionally substituted with one or more of the substituents selected from the group consisting of halogen atom, -OH, -COOH, - ONO₂ or T, wherein T is -OC(O) (C₁-C₁₀ alkyl)-ONO₂ or -O(C₁-C₁₀ alkyl)-ONO₂;
   b) a C₅-C₇ cycloalkylene group optionally substituted with linear or branched C₁-C₁₀ alkyl group;
   c) 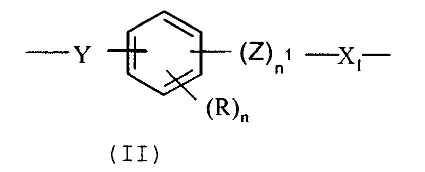 wherein:
      Y is straight or branched C₁-C₂₀ alkylene, or -CH=CH-(CH₂)ₙ²-wherein n² is an integer from 0 to 10;
      R is H, C₁-C₅ alkyl, -COOH, or -OR' wherein R' is H or a C₁-C₃ alkyl group;
      Z is O, -C(O)O- or -OC(O)-;
      n is 0 or 1;
      n¹ is 0 or 1;
      X₁ is a straight or branched C₁-C₂₀ alkylene, optionally substituted with one or more of the substituents selected from the group consisting of halogen atoms, -OH, -COOH, - ONO₂ or X₁ is a group of formula (III): 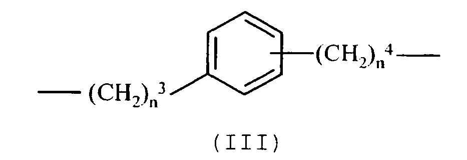
      wherein:
      n³ is an integer from 0 to 5;
      n⁴ is an integer from 1 to 5;
      wherein, the group D of formula (I) is bound to the X₁ group of formula (II), and to the -(CH₂)ₙ⁴- group of formula (III);
   d) 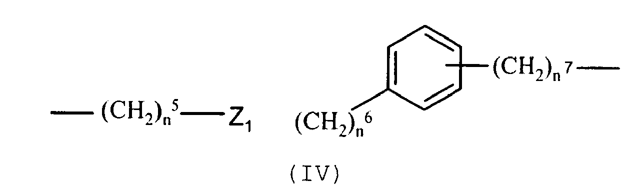
      wherein n⁵ is an integer from 1 to 20;
      Z₁ is -C(O)O- or -OC(O)-;
      n⁶ is an integer from 0 to 20;
      n⁷ is an integer from 1 to 20;
         wherein the group D of formula I) is bound to -(CH₂)ₙ⁷-group;
   e) 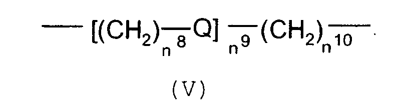
      wherein
      Q is O or S;
      n⁸ is an integer from 1 to 6;
      n⁹ is an integer from 1 to 10;
      n¹⁰ is an integer from 1 to 10;
   f) 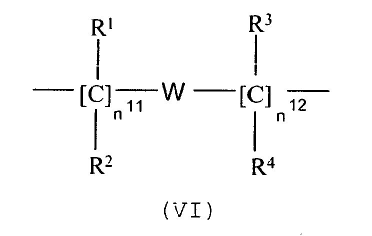 wherein:
      n¹¹ is an integer from 0 to 10;
      n¹² is an integer from 1 to 10;
      R¹, R², R³, R⁴ are the same or different, and they are H or straight or branched C₁-C₄ alkyl;
      wherein the D group of formula (I) is linked to 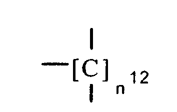 W is an heterocyclic saturated, unsaturated or aromatic 5 or 6 members ring, containing one or more heteroatoms selected from nitrogen, oxygen, sulfur, and is selected from

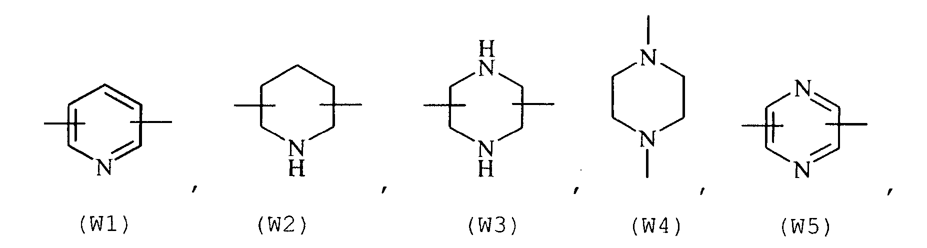

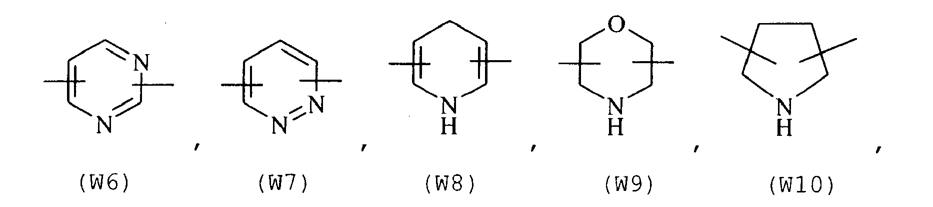

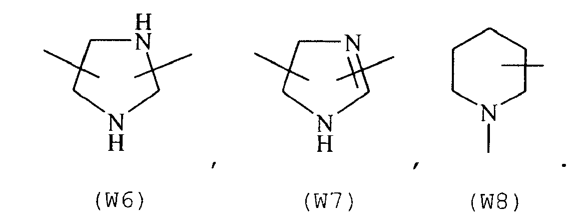

As stated above, the invention includes also the pharmaceutically acceptable salts of the compounds of formula (I) and stereoisomers thereof.

Examples of pharmaceutically acceptable salts are either those with inorganic bases, such as sodium, potassium, calcium and aluminium hydroxides, or with organic bases, such as lysine, arginine, triethylamine, dibenzylamine, piperidine and other acceptable organic amines or bases as those reported for example in Wermuth, C.G. and Stahl, P.H. Pharmaceutical Salts:Properties, Selection, and Use - A Handbook Verlag Helvetica Chimica Acta, 2002 [ISBN 3-906390-26-8].

The compounds according to the present invention, when they contain in the molecule one salifiable nitrogen atom, can be transformed into the corresponding salts by reaction, in an organic solvent such as acetonitrile, tetrahydrofuran, with the corresponding organic or inorganic acids.

Examples of organic acids are: oxalic, tartaric, maleic, succinic, citric acids. Examples of inorganic acids are: nitric, hydrochloric, sulphuric, phosphoric acids. Salts with nitric acid are preferred.

The compounds of the invention which have one or more asymmetric carbon atoms can exist as optically pure enantiomers, pure diastereomers, enantiomers mixtures, diastereomers mixtures, enantiomer racemic mixtures, racemates or .racemate mixtures. Within the scope of the invention are also all the possible isomers, stereoisomers and their mixtures of the compounds of formula (I), including mixtures enriched in a particular isomer.

The term "aryl group" refers to a mono or bicyclic carbocyclic ring system having one or two aromatic rings including phenyl, naphtyl and like. Aryl groups can be unsubstituted or substituted with one, two or three substituents independently selected from branched or straight C₁-C₅ alkyl, haloalkyl, alkoxy, amino, alkylamino, dialkylamino, hydroxyl, halogen atom and nitro.

The term "C₁-C₁₂ alkoxy" as used herein refers to branched or straight chains preferably having from 1 to 10 carbon atoms such as methoxy, ethoxy, n-propoxy, isopropoxy, n-butoxy, isobutoxy, t-butoxy, pentyloxy, hexyloxy, octyloxy and the like.

The term "C₁-C₂₀ alkylene" as used herein refers to branched or straight C₁-C₂₀ hydrocarbon chain, preferably having from 1 to 10 carbon atoms such as methylene, ethylene, propylene, isopropylene, n-butylene, pentylene, n-hexylene and the like.

The term "C₁-C₁₀ alkyl" as used herein refers to branched or straight alkyl groups comprising 1 to 10 carbon atoms, including methyl, ethyl, n-propyl, isopropyl, n-butyl, isobutyl, t-butyl, pentyl, hexyl, octyl and the like.

The term "C₁-C₅" alkyl as used herein refers to branched or straight alkyl groups comprising 1 to 5 carbon atoms, including methyl, ethyl, n-propyl, isopropyl, n-butyl, isobutyl, t-butyl, pentyl, and the like.

The term "C₁-C₄" alkyl as used herein refers to branched or straight alkyl groups comprising 1 to 4 carbon atoms, including methyl, ethyl, n-propyl, isopropyl, n-butyl, isobutyl, t-butyl.

The term "cycloalkylene" as used herein refers to ring having from 5 to 7 carbon atoms including, but not limited to, cyclopentylene, cyclohexylene, optionally substituted with side chains such as straight or branched (C₁-C₁₀)-alkyl, preferably CH₃.

The term "halogen" as used herein refers to fluorine, chlorine, bromine, iodine.

Preferred compounds of formula (I) are those, wherein:
X is:
   a) straight or branched C₁-C₁₀ alkylene, optionally substituted with one or more of the substituents selected from the group consisting of halogen atom, -OH, -COOH, -ONO₂ or T, wherein T is -OC(O)(C₁-C₁₀ alkyl)-ONO₂ or -O(C₁-C₁₀ alkyl)-ONO₂;
   c) 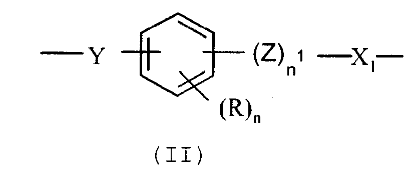 wherein:
      Y is straight or branched C₁-C₆ alkylene, or -CH=CH-(CH₂)ₙ²-wherein and n² is 0 or 1;
      R is H, -CH₃, -COOH, or -OR' wherein R' is H or -CH₃;
      Z is O, -OC(O)-;
      n is 0 or 1;
      n¹ is 0 or 1;
      X₁ is a straight or branched C₁-C₁₀ alkylene, optionally substituted with one or more of the substituents selected from the group consisting of halogen atoms, -OH, -COOH, -ONO₂ or X₁ is a group of formula (III): 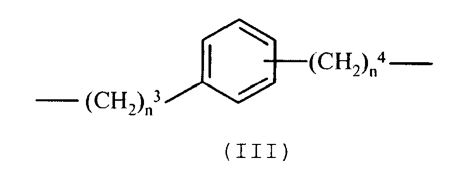
      wherein:
      n³ is 0 or 1;
      n⁴ is 1;
      wherein, the group D of formula (I) is bound to the X₁ group of formula (II), and to the -(CH₂)ₙ⁴- group of formula (III);
   d) 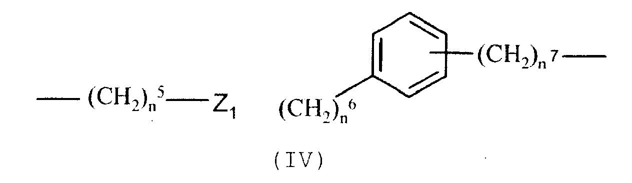 wherein n⁵ is an integer from 1 to 10;
      Z₁ is -C(O)O- or -OC(O)-;
      n⁶ is an integer from 0 to 10;
      n⁷ is an integer from 1 to 10;
      wherein the group D of formula I) is bound to -(CH₂)ₙ⁷-group;
   e) 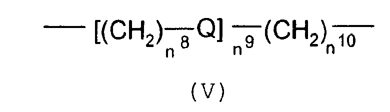 wherein
      Q is -O- or -S-;
      n⁸ is an integer from 1 to 4;
      n⁹ is an integer from 1 to 6;
      n¹⁰ is an integer from 1 to 6;
   f) 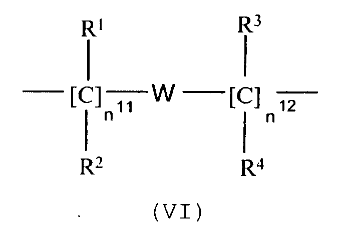 wherein:
      n¹¹ is an integer from 0 to 4;
      n¹² is an integer from 1 to 4;
      R¹, R², R³, R⁴ are H;
      wherein the D group of formula (I) is linked to 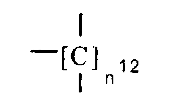
      W is an heterocyclic ring selected from:

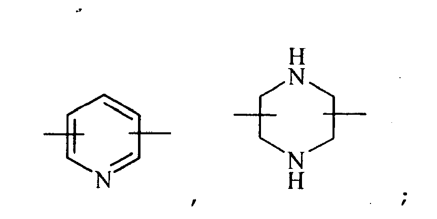

Most preferred compounds of formula (I) are those wherein:
X is:
   a) straight or branched C₁-C₁₀ alkylene, optionally substituted with one or more -ONO₂ groups;
   c) 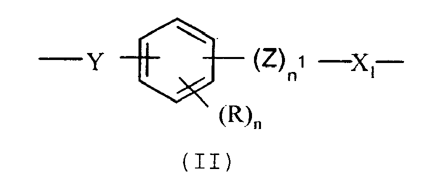 wherein:
      Y is straight or branched C₁-C₆ alkylene, or -CH=CH-(CH₂)ₙ²-wherein and n² is 0 or 1;
      R is H or -OR' wherein R' is CH₃;
      Z is O or -OC (O) -;
      n¹ is 0 or 1;
      X₁ is a straight or branched C₁-C₆ alkylene, optionally substituted with one or more -ONO₂ groups or X₁ is a group of formula (III): 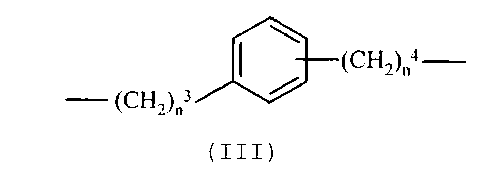
      wherein:
      n³ is 0 or 1;
      n⁴ is 1;
      wherein, the group D of formula (I) is bound to the X₁ group of formula (II), and to the -(CH₂)ₙ⁴- group of formula (III);
   d) 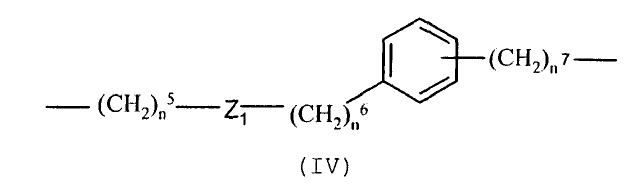 wherein n⁵ is an integer from 1 to 5;
      Z₁ is -C(O)O- or -OC(O)-;
      n⁶ is an integer from 0 to 5;
      n⁷ is an integer from 1 to 5;
      wherein the group D of formula I) is bound to -(CH₂)ₙ⁷-group;
   e) 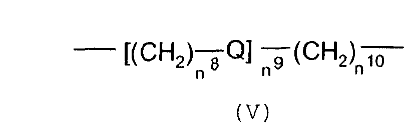 wherein
      Q is O;
      n⁸ is 1 or 2;
      n⁹ is an integer from 1 to 4;
      n¹⁰ is an integer from 1 to 2;

Particularly preferred compounds are compounds of formula(I) according to claim 1 selected from the group:

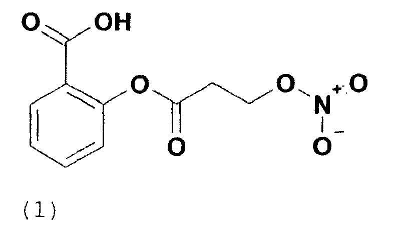

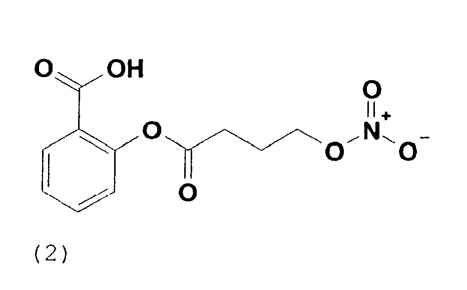

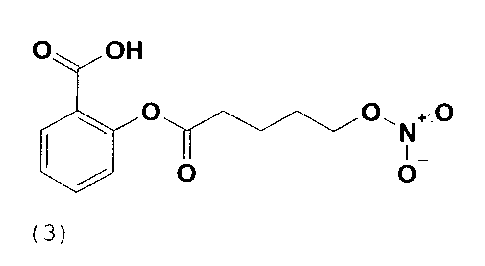

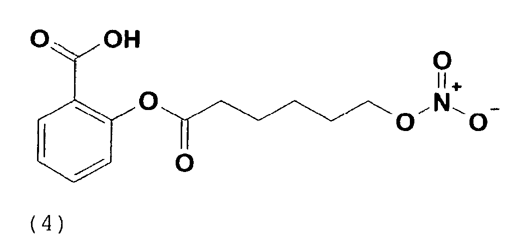

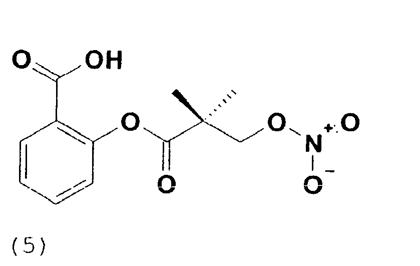

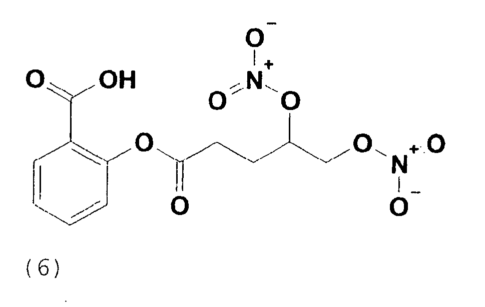

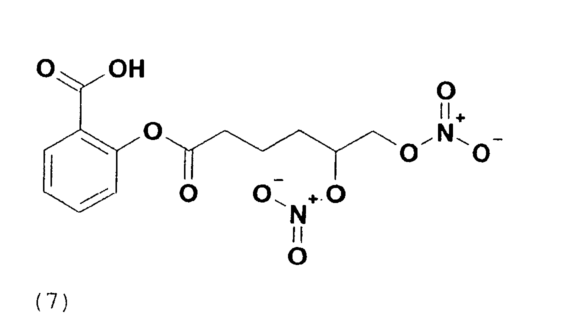

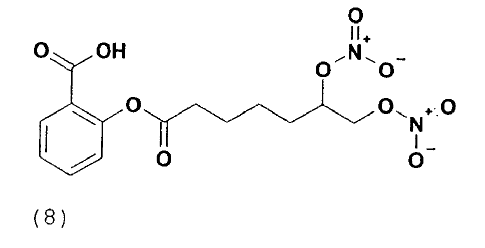

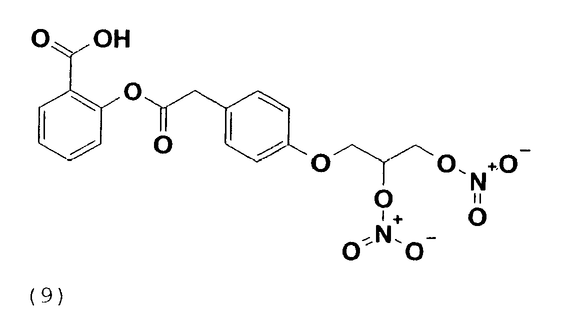

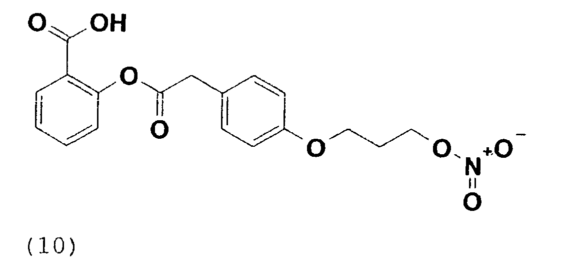

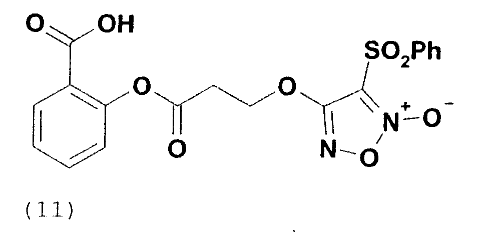

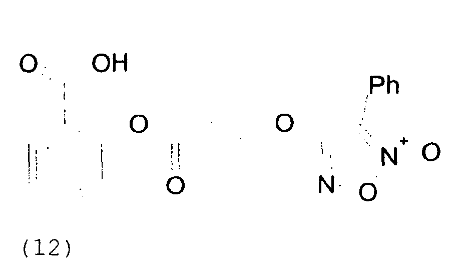

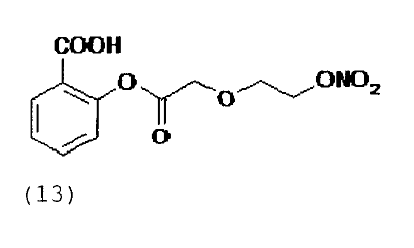

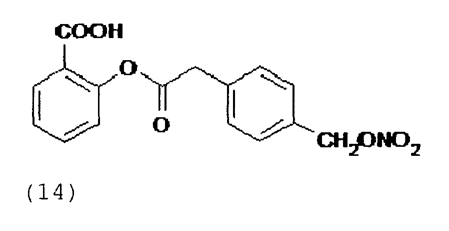

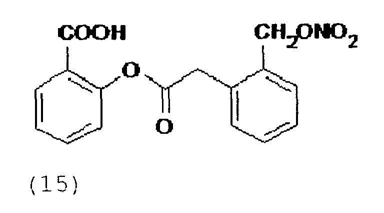

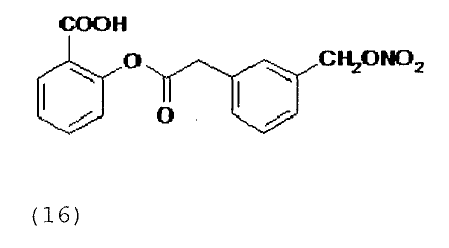

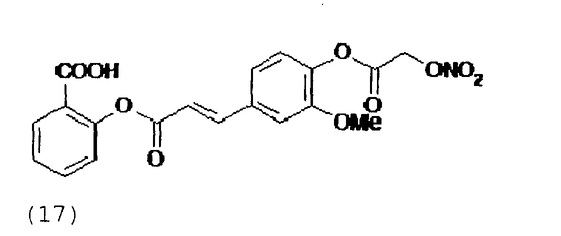

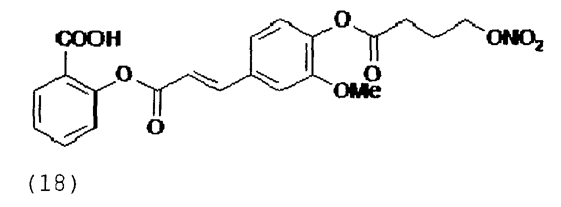

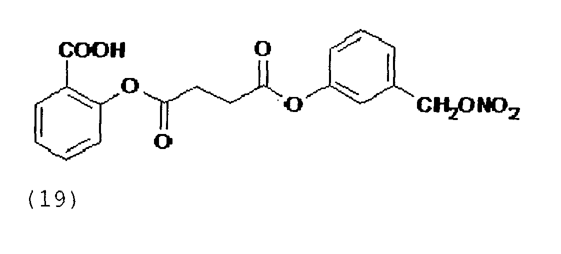

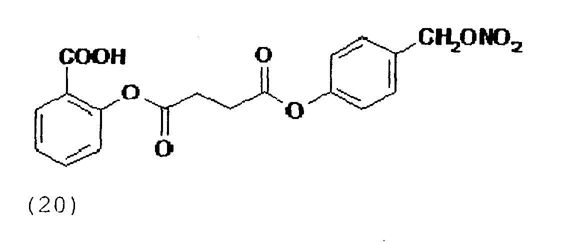

The compounds of formula (I) as above defined can be prepared by a process comprising the oxidation of a compound of formula (VIII): 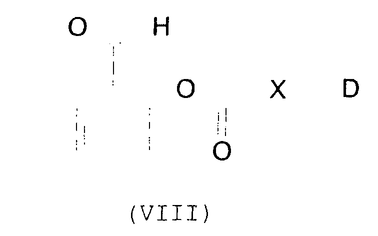 wherein X and D are as defined above.

The oxidation of the aldehyde group to carboxylic acid can be carried out by reacting a compound of formula (VIII) with a suitable oxidising agent such as potassium permanganate, sodium chlorite or sodium chlorite/H₂O₂ in a suitable organic solvent such acetic acid and the like at a temperature from 0 to 80°C for a time from 1 minute to 72 hours.

Compounds of general formula (VIII) in which D is ONO2 can be obtained by nitrating compounds of general formula (IX): 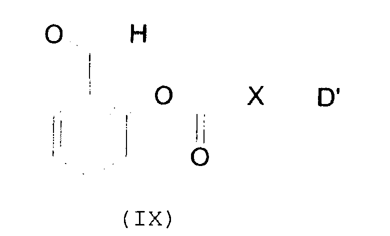 in which D' is chlorine, bromine, iodide, tosylate, mesylate, trifluoromethanesulfonate and the like or OH. When D' is chlorine, bromine, iodide, tosylate, mesylate, trifluoromethanesulfonate and the like, the compound of formula (IX) is reacted with silver nitrate in a suitable aprotic organic solvent such as acetone, tetrahydrofuran, acetonitrile, preferably acetonitrile.

Alternatively compounds of general formula (IX) in which D' is an hydroxyl group can be converted into compounds of general formula (VIII) in which D is ONO₂, by reaction with nitric acid in a suitable solvent, such as acetic acid. Finally, they could be obtained by action of N-Bromosuccinimide (NBS), triphenylphosphine (Ph₃P) and AgNO₃.

Compounds of general formula (IX) can be obtained by reacting ortho salicylic aldehyde with a suitably activated carboxylic acid or acyl halide of formula (X):

D'-X-CO-L (X)

wherein L is halogen or an acyl activating group such as those reported as a mater of example in Comprehensive Organic Transformations : A Guide to Functional Group Preparations by Richard C. Larock second edition 1999, optionally in the presence of a suitable base such as triethylamine, diisopropylethylamine, pyridine in a suitable solvent such as an halogenated solvent such as dichloromethane or 1,2 dichloroethane, or an hydrocarbon such as toluene, chlorobenzene.

Alternatively compounds of formula (VIII) can be obtained by reacting ortho salicylic aldehyde with a suitably activated carboxylic acid or acyl halide of formula (XI):

D-X-CO-L (XI)

wherein D is as defined above, L is halogen or an acyl activating group such as those reported for example in Comprehensive Organic Transformations : A Guide to Functional Group Preparations by Richard C. Larock second edition 1999, following the above reported procedure for the reaction of salicylic aldehyde with the compound of formula (X).

Alternatively the compounds of formula (I) wherein D is ONO₂ by can be obtained by reacting salicylic acid with a compound of formula (XI) as above defined, according the procedure reported above.

Compounds of formula (X) or formula (XI) can be obtained from the corresponding acids of formula (XII) or (XIII) by well known reactions:

D'-X-CO-COOH (XII)

D-X-CO-COOH (XIII)

wherein D' and D are as defined above.

Compounds of formula (XIII) wherein is ONO₂ can be prepared by the compounds of formula (XII) by nitration as above reported for compounds of formula (IX).

Compounds of formula (XI) are commercially available or can be prepared by methods well known in the art.
Compounds of formula (XIII) wherein D is 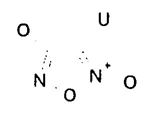 are prepared from the corresponding alcohol by oxidation with Jones reagent in acetone at a temperature between 0°C and 25°C which in turn are prepared according to the procedure reported by Cena et al. Pharm. Res. 2001, 18, 157. Alternatively compounds of formula (I) wherein X is a straight or branched C₁-C₂₀ alkyl substituted by a ONO₂ group having the following formula (XIV):

-X'-(CHONO₂)-CH₂- (XIV)

wherein X' is a straight or branched C₁-C₁₈ alkyl can be prepared by oxidation of a compound of formula (XV):

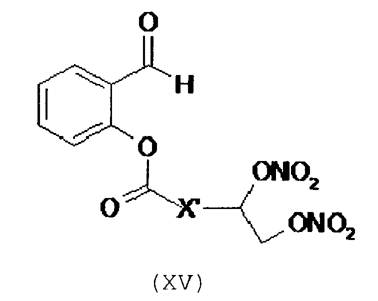

Suitable oxidating agent can be potassium permanganate, sodium chlorite or sodium chlorite/H₂O₂ in a suitable organic solvent such acetic acid and the like at a temperature from 0 to 80°C for a time from 1 minute to 72 hours.

Compounds of formula (XV) can be prepared from compounds of formula (XVI) by treatment with iodine and silver nitrate in acetonitrile at a temperature between -20°C and 80°C.

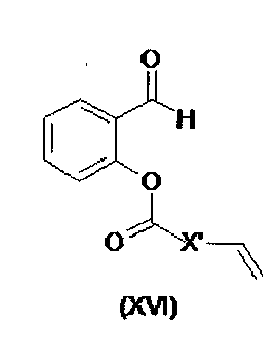

Compounds of formula (XV)can be prepared by reaction of salicylic aldehyde with a compound of formula (XVII): 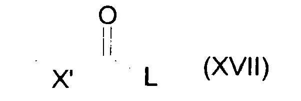 wherein X' and L are as above defined.

Compounds of formula (XVII) can be obtained from the corresponding acids of formula (XVIII):

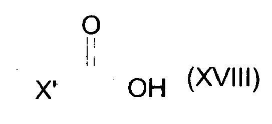

Compounds of formula (XVIII) are known compounds or can be obtained by methods well known in the art.

As mentioned above, object of the present invention are also pharmaceutical compositions containing at least a compound of the present invention of formula (I) together with non toxic adiuvants and/or carriers usually employed in the pharmaceutical field.

The daily dose of active ingredient that should be administered can be a single dose or it can be an effective amount divided into several smaller doses that are to be administered throughout the day. Usually, total daily dose may be in amounts preferably from 50 to 500 mg. The dosage regimen and administration frequency for treating the mentioned diseases with the compound of the invention and/or with the pharmaceutical compositions of the present invention will be selected in accordance with a variety of factors, including for example age, body weight, sex and medical condition of the patient as well as severity of the disease, route of administration, pharmacological considerations and eventual concomitant therapy with other drugs. In some instances, dosage levels below or above the aforesaid range and/or more frequent may be adequate, and this logically will be within the judgment of the physician and will depend on the disease state.

The compounds of the invention may be administered orally, parenterally, rectally or topically, by inhalation or aerosol, in formulations eventually containing conventional non-toxic pharmaceutically acceptable carriers, adjuvants and vehicles as desired. Topical administration may also involve the use of transdermal administration such as transdermal patches or iontophoresis devices. The term "parenteral" as used herein, includes subcutaneous injections, intravenous, intramuscular, intrasternal injection or infusion techniques.

Injectable preparations, for example sterile injectable aqueous or oleaginous suspensions may be formulated according to known art using suitable dispersing or wetting agents and suspending agents. The sterile injectable preparation may also be a sterile injectable solution or suspension in a non-toxic parenterally acceptable diluent or solvent. Among the acceptable vehicles and solvents are water, Ringer's solution and isotonic sodium chloride. In addition, sterile, fixed oils are conventionally employed as a solvent or suspending medium. For this purpose any bland fixed oil may be employed including synthetic mono or diglycerides, in addition fatty acids such as oleic acid find use in the preparation of injectables.

Suppositories for rectal administration of the drug can be prepared by mixing the active ingredient with a suitable non-irritating excipient, such as cocoa butter and polyethylene glycols.

Solid dosage forms for oral administration may include capsules, tablets, pills, powders, granules and gels. In such solid dosage forms, the active compound may be admixed with at least one inert diluent such as sucrose, lactose or starch. Such dosage forms may also comprise, as in normal practice, additional substances other than inert diluents, e.g. lubricating agents such as magnesium stearate. In the case of capsules, tablets and pills, the dosage forms may also comprise buffering agents. Tablets and pills can additionally be prepared with enteric coatings.

Liquid dosage forms for oral administration may include pharmaceutically acceptable emulsions, solutions, suspensions, syrups and elixirs containing inert diluents commonly used in the art, such as water. Such compositions may also comprise adjuvants, such as wetting agents, emulsifying and suspending agents, and sweetening, flavouring and the like.

### Experimental procedures

Melting points were determined on a Büchi 540 apparatus and are uncorrected. The compounds were routinely checked by mass spectrometry (Finnigan-MatTSQ-700 spectrometer, 70 eV, direct inlet). ¹H-, proton decoupled ¹³C-NMR spectra, were recorded on a Bruker AC-300 spectrometer. The following abbreviations are used to indicate peak multiplicity: *s* = singlet; *d* = doublet; *t* = triplet; *qt* = quartet; *qn* = quintet; se = sextet; m = multiplet). Flash column chromatography was performed on silica gel (Merck Kiesekgel 60, 230-400 mesh ASTM). Thin layer chromatography (TLC) was carried out on 5 x 20 cm plates with a layer thickness of 0.25 mm. HPLC on analytical scale was performed using a diode array UV detector (Shimadzu LC10A). HPLC on preparative scale was performed on Varian Prostar. Eluents are indicated in the synthetic procedure.

### Example 1

### Method a

### 2-{[3-(nitrooxy)propanoyl]oxy}benzoic acid (compound 1)

SOCl₂ (2.43 mL, 33.3 mmol) and a few drops of dry DMF were added to a solution of 3-(nitrooxy)propionic acid (3.0 g, 22.2 mmol; J. Org. Chem. 1956, 21, 367-368) in dry THF (20 mL), stirred under N₂ at r.t.. The stirring was continued for 3 h at r.t. The solution of the acyl chloride so obtained was slowly added to a stirred solution of salycilic acid (3.07 g, 22.2 mmol) and dry Pyridine (2.7 mL, 33.3 mmol) in dry THF (40 mL), kept under N₂ at 0 °C. The mixture was allowed to reach r.t. and the stirring was continued overnight. The mixture was diluted with Et₂O (90 mL) and washed twice with HCl 2M (60 mL). The organic layer was dried with MgSO₄, filtered and concentrated under reduced pressure. The crude product was partially purified by flash chromatography (CH₂Cl₂/MeOH 97/3 v/v). The solid product (2g) so obtained was crystallised from toluene.
Yield 46 %.
mp 86-88°C (from toluene)
TLC: Rf= 0.45 PE/EtOAc/HCOOH 70/30/01 v/v/v

### Method b

### 2-formylphenyl 3-(nitrooxy)propionate

SOCl₂ (2.53 mL, 34.6 mmol) and a few drops of dry DMF were added to a solution of 3-(nitrooxy)propionic acid (3.9 g, 28.9 mmol) in dry CH₂Cl₂ (40 mL), stirred under N₂ at r.t. The stirring was continued for 2 h at r.t. The solution of the acyl chloride so obtained was slowly added to a stirred solution of salicylic aldehyde (2.5 ml, 23.1 mmol) and dry Pyridine (3.5 mL, 43.3 mmol) in dry CH₂Cl₂ (40 mL), kept under N₂ at 0 °C. The mixture was allowed to reach room temperature and the stirring was continued for 5 h. The mixture was washed with 2M HCl (3 x 60 mL) and the combined organic layers were dried over MgSO₄, filtered and concentrated under reduced pressure. The crude product so obtained was purified by flash chromatography (PE/EtOAc 90/10 v/v) to give the **title compound** as pale yellow oil (1.3g).
Yield 23 %.
TLC: Rf= 0.48 PE/EtOAc 80/20 v/v

**2-{[3-(nitrooxy)propanoyl]oxy)}benzoic acid** (compound 1) CH₃COOH (72 µL) and NaClO₂ (0.40 g, 4.38 mmol) were added to a stirred solution of 2-formylphenyl 3-(nitrooxy)propanoate (0.30 g, 1.25 mmol) in CH₂Cl₂ (13 mL). The mixture was stirred at r.t. for 24h, then was washed twice with H₂O (10 mL), dried with MgSO4, filtered and concentrated under reduced pressure. The crude product so obtained was crystallized by PE/toluene 40/60 v/v to give the **title compound** as white solid (33 mg).
Yield 30%,
mp 86-88°C (PE/toluene 40/60 v/v)
TLC: Rf= 0.45 PE/EtOAc/HCOOH 70/30/01 v/v/v

¹H-NMR (CDCl₃) δ 3. 09 (2H, t, J = 6. 4 Hz), 4.87 (2H, t , J = 6.4H₂), 7.16 (1H, d, Arom), 7.39 (1H, t, Arom), 7.65 (1H, t, Arom), 8.16 (1H, d, Arom), 10.0 (1H, s vvbr). ¹³C-NMR (CDCl₃) δ 32.2, 67.6, 121.8, 123.9, 126.6, 132.7, 135.2, 150.8, 168.3, 169.8. MS (CI) *m*/*z* 256 (M+1)⁺.

### Example 2

### Method a

**2-{[5,6-bis(nitrooxy)hexanoyl]oxy}benzoic acid** (compound 7) SOCl₂ (370 µL, 5.04 mmol) and a few drops of dry DMF were added to a solution of 5,6-bis(nitrooxy)hesanoic acid (1.00 g, 4.20 mmol; Lazzarato L. et al. J. Med Chem. 2005, 48(5), 1322) in dry THF (20 mL), stirred under N₂ at r.t. The stirring was continued for 6 hrs at r.t. The solution of the acyl chloride so obtained was slowly added to a stirred solution of salycilic acid (0.58 g, 4.20 mmol) and dry Pyridine (510 µL, 6.30 mmol)in dry THF (20 mL), kept under N₂ at 0 °C. The mixture was allowed to reach r.t. and then stirred overnight. The mixture was diluted with Et₂O (50 mL) and washed twice with 2M HCl (45 mL). The combined organic layers were dried with MgSO₄, filtered and concentrated under reduced pressure. The crude product so obtained was purified by preparative HPLC (Lichrospher 250-25 C₁₈, CH₃CN/H₂O/TFA 50/50/0.1, flow 39 mL/min, λ 224 nm, injection 2.5 mL, solution 75 mg/mL) to give the **title compound** as white solid (472 mg).
Yield 31 %.
m.p. 101.5-102.5°C (from toluene).
TLC: Rf= 0.44 PE/EtOAc/HCOOH 70/30/01 v/v/v

### Method b

### 2-formylphenyl 5,6-bis(nitrooxy)hexanoate

SOCl₂ (477 µL, 6.55 mmol) and a few drops of dry DMF were added to a solution of 5,6-bis(nitrooxy)hexanoic acid (1.30 g, 5.46 mmol) in dry CH₂Cl₂ (15 mL), stirred under N₂ at r.t. The stirring was continued for 2 hrs at r.t. The solution of the acyl chloride so obtained was slowly added to a stirred solution of salicylaldehyde (465 µL, 4.37 mmol) and dry Pyridine (660 µL, 8.19 mmol) in dry CH₂Cl₂ (10 mL), kept under N₂ at 0 °C. The reaction was allowed to reach r.t. and then stirred for 2.5 hrs. Then the mixture was washed with 2M HCl (3 x 12 mL). The combined organic layers were dried with MgSO₄, filtered and concentrated under reduced pressure. The crude product so obtained was purified by flash chromatography (PE/EtOAc 80/20 v/v) to give the **title compounds** as pale yellow oil (1g).
Yield 56 %.
TLC: Rf= 0.69 PE/EtOAc 80/20 v/v

### 2-{[5,6-bis(nitrooxy)hexanoyl]oxy}benzoic acid (compound 7)

KMnO₄ (0.69 g, 4.38 mmol) was added to a stirred solution of 2-formylphenyl 5,6-bis(nitrooxy)hexanoate (1.00 g, 2.92 mmol) in acetone (20 mL) kept at 0 °C. The reaction was allowed to reach r.t. and was completed after 3h (TLC detection, eluent Petrol ether/EtOAc 70/30 v/v). Oxalic acid was added and the mixture was filtered and the filtrate was diluted with CH₂Cl₂ (20 mL). The organic layer was washed with H₂O (20 mL) and then was dried with MgSO₄, filtered and concentrated under reduced pressure. The crude product was crystallized with PE/Toluene 50/50 v/v to give the **title compound** as white solid (580 mg).
Yield 72 %.
m.p. 101.5-102.5°C from PE/Toluene 50/50 v/v.
TLC. Rf=0.44 PE/EtOAc/HCOOH 70/30/01 v/v/v

¹H-NMR (DMSO-d₆) δ 1.73-1.86 (4H, m), 2.64 (2H, t , J = 6.0H₂), 4.73 (1H, dd, AMX like system), 4.96 (1H, dd, AMX like system) , 5.46 (1H, m, AMX like system), 7.19 (1H, d, Arom ), 7.39 (1H, t, Arom ), 7.64 (1H, t, Arom ), 7.93 (1H, d, Arom), 13.3 (1H, s br). ¹³C-NMR (DMSO-d₆) δ 19.5, 27.5, 32.8, 71.7, 80.0, 123.7, 123.9, 126.0, 131.3, 133.7, 150.0, 165.5, 171.2. MS (CI) *m*/*z* 359 (M+1)⁺.

### Example 3

### 3-{[5-oxido-4-(phenylsulfonyl)-1,2,5-oxadiazol-3-yl]oxy}propanoic acid

A solution of Jones reagent 2.5 M (19 mL, 46.62 mmol) was added to a stirred solution of 3-{[5-oxido-4-(phenylsulfonyl)-1,2,5-oxadiazol-3-yl]oxy}propan-1-ol (5.6 g, 18.65 mmol; Cena et al. Pharm. Res. 2001, 18, 157) in acetone (150 mL), cooled at 0 °C. The mixture was allowed to reach r.t. and stirred for 4h. iPrOH (10 mL) was added and the mixture was concentrated under reduced pressure. The residue was dissolved with EtOAc (150 mL) and was extracted with a saturated solution of NaHCO₃ (3 x 20 mL). The aqueous layers were acidified with HCl 6M and extracted twice with EtOAc (50 mL). The combined organic layers were dried with MgSO₄, filtered and concentrated under reduced pressure to give the **title compound** as white solid (3.64 g).
Yield 65 %.
m.p. 142-143°C (from toluene).
TLC: Rf= 0.38 CH₂Cl₂/EtOAc 95/5 v/v

¹H-NMR (DMSO-d₆) δ 2.81 (2H, *t,* J = 5.8 Hz), 4.59 (2H, *t,* J = 5.8 Hz), 7.72-8.01 (5H, *m,* Arom ), 12.63 (1H, *s br* ). ¹³C-NMR (DMSO-d₆) δ 33.3, 67.4, 110.5, 128.3, 130.1, 136.3, 137.3, 158.8, 171.5 . MS (CI) *m*/*z* 315 (M+1)⁺.

### 2-[(3-{[5-oxido-4-(phenylsulfonyl)-1,2,5-oxadiazol-3-yl]oxy}propanoyl)oxy]benzoic acid (compound 11)

SOCl₂ (334 µL, 4.58 mmol) and a few drops of dry DMF were added to a solution of 3-{[5-oxido-4-(phenylsulfonyl)-1,2,5-oxadiazol-3-yl]oxy}propanoic acid (1.20 g, 3.82 mmol) in dry THF (20 mL), stirred under N₂ at r.t. The stirring was continued for 7 h at r.t. The solution of the acyl chloride so obtained was slowly added to a stirred solution of salycilic acid (0.53 g, 3.82 mmol) and dry Py (463 µL, 5.73 mmol) in dry THF (20 mL) kept under N₂ at 0 °C. The mixture was allowed to reach r.t. and then stirred overnight. The mixture was diluted with Et₂O (50 mL) and washed twice with HCl 2M (50 mL). The combined organic layers were dried with MgSO₄, filtered and concentrated under reduced pressure. The crude product was purified by preparative HPLC (Lichrospher 250-25 C₁₈, CH₃CN/H₂O/TFA 50/50/0.1, flow 39 mL/min, λ 224 nm, injection 4 mL, solution 51 mg/mL) to give the title compound as white solid.
Yield 32 %.
mp 169 - 170°C (from toluene).
TLC: Rf = 0.27 PE/EtOAc/HCOOH 60/40/0.1 v/v/v
¹H-NMR (DMSO-d₆) δ 3.17 (2H, *t,* J = 6.0 Hz), 4.74 (2H, *t ,* J = 6.0 Hz), 7.23-8.01 (9H, *m,* Arom), 13.16 (1H, *s br*). ¹³C-NMR (DMSO-d₆) δ 33.3, 66.6, 110.4, 123.6, 123.7, 126.3, 128.1, 129.8, 131.5, 133.9, 136.0, 137.1, 149.8, 158.6, 165.4, 168.6 , MS (CI) *m*/*z* 435 (M+1)⁺.

### Example 4

### 2-formylphenyl 4-bromobutanoate

SOCl₂ (1.60 mL, 21.6 mmol) and a few drops of dry DMF were added to a solution of 4-bromobutyrric acid (3.00 g, 18.0 mmol) in dry CH₂Cl₂ (40 mL), stirred under N₂ at r.t. The stirring was continued for 3 h at r.t. The solution of the acyl chloride so obtained was slowly added to a stirred solution of salycilic aldehyde (1.73 mL, 14.4 mmol) and dry Pyridine (2.20 mL, 27.0 mmol) in dry CH₂Cl₂ (40 mL), kept under N₂, at 0 °C. The reaction was allowed to reach r.t. and was completed after 2 h. The mixture was washed with HCl 2M (3 x 30 mL). The organic layer was dried with MgSO₄, filtered and concentrated under reduced pressure. The crude product was purified by flash chromatography (PE/EtOAc 90/10 v/v) to give the **title compound** as pale yellow oil (3.54g).
Yield 80 %.
TLC: Rf = 0.67 PE/EtOAc 80/20 v/v

¹H-NMR (CDCl₃) δ 2.34-2.36 (2H, *m*), 2.87-2.92 (2H, *m*), 3.55-3.60 (2H, *m*), 7.18 (1H, *d,* Arom), 7.43 (1H, *t,* Arom ), 7.66 (1H, *t,* Arom), 7.88 (1H, *d,* Arom), 10.1 (1H, *s br*). ¹³C-NMR (CDCl₃) δ 27.4, 32.3, 32.5, 123.5, 126.5, 132.0, 134.8, 151.0, 171.0, 188.9. MS (CI) *m*/*z* 271/273 (M+1)⁺.

### 2-formylphenyl 4-(nitrooxy)butanoate

A solution of 2-formylphenyl 4-bromobutanoate (5.00 g, 18.4 mmol) and AgNO₃ (7.83 g, 46.0 mmol) in CH₃CN (150 mL) was stirred at 70 °C for 7h. The mixture was filtered and concentrated under reduced pressure. The residue was treated with CH₂Cl₂ (50 mL) and H₂O (50 mL). After separation the aqueous layer was extracted twice with CH₂Cl₂ (50 mL). The combined organic layers were dried with MgSO₄, filtered and concentrated under reduced pressure. The crude product was purified by flash chromatography (PE/EtOAc 90/10 v/v) to give the **title compound** as pale yellow oil (3.88 g).
Yield 84 %.
TLC: Rf = 0.50 PE/EtOAc 80/20 v/v

¹H-NMR (CDCl₃) δ 2.17-2.27 (2H, *m*), 2.82 (2H, *t*, J = 7.1 Hz), 4.61 (2H, *t,* J = 6.2 Hz), 7.18 (1H, *d,* Arom), 7.44 (1H, *t,* Arom), 7.65 (1H, *t,* Arom), 7.87 (1H, *d,* Arom), 10.0 (1H, *s*). ¹³C-NMR (CDCl₃) δ 22.1, 30.1, 71.8, 123.5, 126.7, 132.7, 134.9, 150.6, 170.8, 189.1. MS (CI) *m*/*z* 254 (M+1)⁺.

### 2-{[4-(nitrooxy)butanoyl]oxy}benzoic acid (compound 2)

KMnO₄ (3.28 g, 20.7 mmol) was added to a stirred solution of 2-formylphenyl 4-(nitrooxy)butanoate (3.5 g, 13.8 mmol) in acetone (100 mL), kept at 0 °C. The reaction was allowed to reach r.t. and it was completed after 3h (TLC detection, eluent PE/EtOAc 70/30 v/v). Oxalic acid was added and the mixture was filtered and the filtrate was diluted with CH₂Cl₂ (50 mL). The organic layer was washed with H₂O (50 mL) and brine (50 mL) and then it was dried with MgSO₄, filtered and concentrated under reduced pressure. The crude product so obtained was crystallised with PE/toluene 50/50 v/v to give the **title compound** as white solid (1.93 g).
Yield 52 %.
m.p. 70.5 - 71.5 °C (from PE/toluene 50/50 v/v).
TLC: Rf = 0.41 PE/EtOAc/HCOOH 60/40/0.1 v/v/v

¹H-NMR (DMSO-d₆) δ 2.05 (2H, *q,* J = 6.0 Hz), 2.71 (2H, *t,* J = 6.0 Hz), 4.63 (1H, *d,* J = 6.0 Hz), 7.21 (1H, *d,* Arom), 7.39 (1H, *t,* Arom), 7.65 (1H, *t,* Arom), 7.94 (1H, *d,* Arom ), 13.13 (1H, *s*). ¹³C-NMR (DMSO-d₆) δ 21.4, 29.7, 72.6, 123.7, 123.8, 126.1, 131.3, 133.8, 150.0, 165.5, 170.9 . MS (CI) *m*/*z* 270 (M+1)⁺.

### Example 5

### 2-formylphenyl 5-bromopentanoate

SOCl₂ (1.45 mL, 19.9 mmol) and a few drops of dry DMF were added to a solution of 5-bromopentanoic acid (3.0 g, 16.6 mmol; J. Am. Chem. Soc. 1947, 69, 24.00) in dry CH₂Cl₂ (20 mL), stirred under N₂ at r.t and the stirring was continued for 1 h . The solution of the acyl chloride so obtained was slowly added to a solution of salycilic aldehyde (1.60 mL, 13.3 mmol) and dry Py (2.00 mL, 24.9 mmol) in dry CH₂Cl₂ (30 mL), stirred at 0 °C under N₂. The reaction was allowed to reach r.t. and it was completed after 2 h. The mixture was washed twice with HCl 2M (30 mL). The organic layer was dried with MgSO₄, filtered and concentrated under reduced pressure. The crude product was purified by flash chromatography (PE/EtOAc 90/10 v/v) to give the **title compound** as pale yellow oil(2.66 g).
Yield 57 %.
TLC: Rf = 0.56 PE/EtOAc 80/20 v/v

¹H-NMR (CDCl₃) δ 1.91-2.05 (4H, *m*), 2.70 (2H, *t,* J = 6.8 Hz), 3.48 (2H, *t,* J = 6.3 Hz), 7.18 (1H, *d,* Arom), 7.40 (1H, *t,* Arom), 7.64 (1H, *t,* Arom), 7.88 (1H, *d,* Arom), 10.1 (1H, *s*). ¹³C-NMR (CDCl₃) δ 23.2, 31.9, 32.9, 33.1, 123.5, 126.5, 128.2, 131.7, 135.3, 151.3, 171.4, 188.8. MS (CI) *m*/*z* 285/287 (M+1)⁺.

### 2-formylphenyl 5-(nitrooxy)pentanoate

A solution of 2-formylphenyl 5-bromopentanoate (2.66 g, 9.33 mmol) and AgNO₃ (4.75 g, 23.3 mmol) in CH₃CN (100 mL) was stirred at 70 °C for 4h. The mixture was filtered and concentrated under reduced pressure. The residue was dissolved with CH₂Cl₂ (50 mL) and H₂O (50 mL). After separation the aqueous layer was extracted twice with CH₂Cl₂ (50 mL). The combined organic layers were dried with MgSO₄, filtered and concentrated under reduced pressure. The crude product was purified by flash chromatography (Petrol ether/EtOAc 90/10 v/v) to give the **title compound** as pale yellow oil (1.9 g).
Yield 64 %.
TLC: Rf = 0.46 PE/EtOAc 80/20 v/v
¹H-NMR (CDCl₃) δ 1.88-1.95 (4H, *m*) , 2. 75 (2H, *t*), 4.55 (2H, *t*), 7.19 (1H, *d*, Arom), 7.43 (1H, *t*, Arom), 7.66 (1H, *t*, Arom), 7.89 (1H, *d*, Arom), 10.1 (1H, *s*). ¹³C-NMR (CDCl₃) δ 20.9, 26.2, 33.3, 72.7, 123.5, 126.5, 128.0, 132.2, 135.4, 151.0, 171.2, 188.9. MS (CI) *m*/*z* 268 (M+1)⁺.

### 2-{[5-(nitrooxy)pentanoyl]oxy}benzoic acid (compound 3)

KMnO₄ (1.68 g, 10.7 mmol) was added, to a solution of 2-formylphenyl 5-(nitrooxy)pentanoate (1.90 g, 7.11 mmol) in acetone (50 mL), stirred at 0 °C. The reaction was allowed to reach r.t. and it was completed after 1h (TLC detection, eluent Petrol ether/EtOAc 70/30 v/v). Oxalic acid was added and the mixture was filtered and the filtrate was diluted with CH₂Cl₂ (50 mL). The organic layer was washed with H₂O (50 mL) and brine (50 mL) and then was dried with MgSO₄, filtered and concentrated under reduced pressure. The crude product was crystallized with PE/toluene 70/30 v/v to give the **title compound** as white solid (1.12 g).
Yield 56 %.
m.p. 48.5-50.5 °C (from PE/toluene 70/30 v/v).
TLC: Rf = 0.40 PE/EtOAc/HCOOH 70/30/0.1 v/v/v

¹H-NMR (CDCl₃) δ 1.89 (4H, *m*), 2.66 (2H, *m*), 4.47 (2H, *m*), 7.12 (1H, *d*, Arom), 7.37 (1H, *t*, Arom), 7.63 (1H, *t*, Arom ), 8.12 (1H, *d*, Arom), 12.1 (1H, *s br*). ¹³C-NMR (CDCl₃) δ 20.9, 26.3, 33.5, 73.0, 122.2, 124.1, 126.4, 132.7, 135.2, 151.3, 170.5, 171.7. MS (CI) *m*/*z* 284 (M+1)⁺.

### Example 6

### 2-formylphenyl 6-bromohexanoate

SOCl₂ (1.35 mL, 18.5 mmol) and a few drops of dry DMF were added to a solution of 6-bromohexanoic acid (3.0 g, 15.4 mmol) in dry CH₂Cl₂ (20 mL) stirred under N₂ at r.t. The stirring was continued for 3h at r.t. The solution of acyl chloride so obtained was slowly added to a solution of salycilic aldehyde (1.64 mL, 12.3 mmol) and dry Py (1.90 mL, 23.1 mmol) in dry CH₂Cl₂ (30 mL), stirred at 0 °C under N₂. The reaction was allowed to reach r.t. and it was completed after 1.5h. The mixture was washed with HCl 2M (3 x 30 mL). The combined organic layers were dried with MgSO₄, filtered and concentrated under reduced pressure. The crude product was purified by flash chromatography (PE/EtOAc 90/10 v/v) to give the title compound as pale yellow oil(3.36 g).
Yield 68 %.
TLC: Rf = 0.59 PE/EtOAc 80/20 v/v

¹H-NMR (CDCl₃) δ 1.57-1.65 (2H, *m*), 1.78-1.83 (2H, *m*), 1.89-1.97 (2H, *m*), 2.70 (2H, *t*, J = 7.5 Hz), 3.44 (2H, *t*, J = 6.7 Hz), 7.19 (1H, *d*, Arom), 7.39 (1H, *t*, Arom), 7.64 (1H, *t*, Arom), 7.88 (1H, *d*, Arom), 10.10 (1H, *s*). ¹³C-NMR (CDCl₃) δ 23.8, 27.6, 32.4, 33.5, 34.0, 123.5, 126.4, 128.1, 131.0, 135.3, 151.5, 171.7, 188.8. MS (CI) *m*/*z* 299/301 (M+1)⁺.

### 2-formylphenyl 6-(nitrooxy)hexanoate

A solution of 2-formylphenyl 6-bromohexanoate (3.2 g, 10.6 mmol) and AgNO₃ (5.4 g, 26.5 mmol) in CH₃CN (100 mL) was stirred at 70 °C for 4h. The mixture was filtered and concentrated under reduced pressure. The residue was dissolved with CH₂Cl₂ (50 mL) and H₂O (50 mL). After separation the aqueous layer was extracted twice with CH₂Cl₂ (50 mL). The combined organic layers were dried with MgSO₄, filtered and concentrated under reduced pressure. The crude product was purified by flash chromatography (PE/EtOAc 90/10 v/v) to give the title compound as pale yellow oil (2.63 g).
Yield 80 %.
TLC: Rf = 0.54 PE/EtOAc 80/20 v/v

¹H-NMR (CDCl₃) δ 1.52-1.62 (2H, *m*), 1.77-1.89 (4H, *m*), 2.68 (2H, *t ,* J = 7.4 Hz), 4.49 (2H, *t,* J = 6.5 Hz), 7.17 (1H, *d,* Arom), 7.41 (1H, *t,* Arom), 7.64 (1H, *t,* Arom), 7.88 (1H, *d,* Arom) , 10.10 (1H, s). ¹³C-NMR (CDCl₃) δ 24.1, 25.1, 26.5, 33.7, 73.0, 123.5, 126.5, 128.1, 131.7, 135.3, 151.3, 171.6, 188.9. MS (CI) *m*/*z* 282 (M+1)⁺.

### 2-{[6-(nitrooxy)hexanoyl]oxy}benzoic acid (compound 4)

KMnO₄ (1.9 g, 12.3 mmol) was added to a solution of 2-formylphenyl 6-(nitrooxy)hexanoate (2.3 g, 8.18 mmol) in acetone (70 mL), stirred at 0 °C,. The reaction was allowed to reach r.t. and it was completed after 2h (TLC detection, eluent PE/EtOAc 80/20 v/v). Oxalic acid was added and the mixture was filtered and the filtrate was diluted with CH₂Cl₂ (100 mL). The organic layer was washed with H₂O (50 mL) and brine (50 mL), and then was dried with MgSO₄, filtered and concentrated under reduced pressure. The crude product was crystallized with PE/toluene 75/25 v/v to give the title compound as white solid (1.9 g).
Yield 82 %.
m.p. 68.0-70.0 °C (from PE/toluene 75/25 v/v).
TLC: Rf = 0.40 PE/EtOAc/HCOOH 70/30/0.1 v/v/v

¹H-NMR (DMSO-d₆) δ 1.91-1.77 (6H, *m*), 2.60 (2H, *t ,* J = 7.3 Hz), 4.55 (2H, *t,* J = 6.0 Hz), 7.20 (1H, *d,* Arom), 7.39 (1H, *t,* Arom), 7.65 (1H, *t,* Arom), 7.94 (1H, *d,* Arom), 13.10 (1H, *s*). ¹³C-NMR (DMSO-d₆) δ 23.5, 24.4, 25.7, 33.1, 73.6, 123.7, 124.1, 126.0, 131.3, 133.7, 150.0, 165.6, 171.5. MS (CI) *m*/*z* 286 (M+1)⁺.

### Example 7

### 2-formylphenyl 2,2-dimethyl-3-(nitrooxy)propanoate

SOCl₂ (530 µL, 7.20 mmol) and a few drops of dry DMF were added to a solution of 2,2-dimethyl-3-(nitrooxy)propanoic acid (0.98 g, 6.00 mmol; Arch. Pharm. Pharm. Med. Chem. 2002, 8, 363-366) in dry CH₂Cl₂ (10 mL), stirred under N₂ at r.t. The stirring was continued over one week. The solution of the acyl chloride so obtained was slowly added to a solution of salycilic aldehyde (640 µL, 4.80 mmol) and dry Py (730 µL, 9.00 mmol) in dry CH₂Cl₂ (12 mL), stirred at 0 °C under N₂. The reaction was allowed to reach r.t. and it was completed after 1 week. The mixture was washed with HCl 2M (3 x 10 mL). The combined organic layers were dried with MgSO₄, filtered and concentrated under reduced pressure. The crude product was purified by flash chromatography (PE/EtOAc 90/10 v/v) to give the title compound as pale yellow oil (800 mg).
Yield 45 %.
TLC: Rf = 0.34 PE/EtOAc 90/10 v/v

### 2-{[2,2-dimethyl-3-(nitrooxy)propanoyl]oxy}benzoic acid (compound 5)

KMnO₄ (0.71 g, 4.48 mmol) was added to a solution of 2-formylphenyl 2,2-dimethyl-3-(nitrooxy)propanoate (0.80 g, 2.99 mmol) in acetone (30 mL) stirred at 0 °C. The reaction was allowed to reach r.t. and it was completed after 4h (TLC detection eluent PE/EtOAc 70/30 v/v). Oxalic acid was added and the mixture was filtered and the filtrate was diluted with CH₂Cl₂ (40 mL). The organic layer was washed with H₂O (20 mL) and brine (20 mL) and then was dried with MgSO₄, filtered and concentrated under reduced pressure. The crude product was crystallized with PE/toluene 75/25 v/v to give the title compound as white solid (371 mg). Yield 61 %.
m.p. 95.0-96 °C (from PE/toluene 75/25 v/v).
TLC: Rf = 0.58 PE/EtOAc/HCOOH 70/30/0.1 v/v/v

¹H-NMR (CDCl₃) δ 1.47 (6H, *s*), 4.67 (2H, *s*), 7.10 (1H, *d,* Arom), 7.37 (1H, *t,* Arom), 7.64 (1H, *t,* Arom), 8.13 (1H, *d,* Arom), 12.1 (1H, *s vvbr*). ¹³C-NMR (CDCl₃) δ 22.3, 42.5, 77.5, 122.2, 123.8, 126.5, 132.6, 135.1, 150.9, 170.2, 172.8. MS (CI) *m*/*z* 284 (M+1)⁺.

### Example 8

### 2-formylphenyl pent-4-enoate

30Cl₂ (2.6 mL, 35.3 mmol) and a few drops of dry DMF were added to a stirred solution of 4-pentenoic acid (3.0 mL, 29.4 mmol) in dry CH₂Cl₂ (20 mL) kept under N₂ at r.t. The stirring was continued for 3h at r.t. The solution of the acyl chloride so obtained was slowly added to a stirred solution of salycilic aldehyde (3.1 mL, 23.5 mmol) and dry Py (3.6 mL, 44.1 mmol) in dry CH₂Cl₂ (30 mL) kept at 0 °C under N₂. The temperature was allowed to reach r.t and the reaction was completed after 1h. The mixture was washed with HCl 2M (3 x 30 mL). The combined organic layers were dried with MgSO₄, filtered and concentrated under reduced pressure. The crude product was purified by flash chromatography (PE/EtOAc 95/5 v/v) to give the title compound as pale yellow oil (4.1 g).
Yield 65 %.
TLC: Rf = 0.52 PE/EtOAc 90/10 v/v

### 2-formylphenyl 4,5-bis(nitrooxy)pentanoate

Iodine (2.48 g, 9.79 mmol) was added portion wise to a stirred solution of 2-formylphenyl pent-4-enoate (2.0 g, 9.79 mmol) and AgNO₃ (1.66 g, 9.79 mmol) in CH₃CN (100 mL) kept at -15 °C. At the end of the addition the stirring was continued for 1h. Then AgNO₃ (3.32 g, 19.6 mmol) was added and the mixture was heated at 70 °C for 20 h. After cooling the mixture was filtered through Celite^{®}. The filtrate was concentrated under reduced pressure, dissolved in water (50 mL) and extracted with EtOAc (4 x 50 mL). The combined organic layers were dried with MgSO₄, filtered and concentrated under reduced pressure. The crude product was purified by flash chromatography (PE/EtOAc 90/10 v/v) to give the title compound as pale yellow oil (2.1 g).
Yield 53 %.
TLC: Rf = 0.47 PE/EtOAc 80/20 v/v

¹H-NMR (CDCl₃) δ 2.10-2.27 (2H, *m*), 2.87 (2H, *t*, J = 6.9 Hz), 4.57 (1H, *dd,* AMX like system), 4.86 (1H, *dd,* AMX like system), 5.52 (1H, *m*), 7.17 (1H, *d,* Arom), 7.46 (1H, *t,* Arom), 7.64 (1H, *t,* Arom), 7.85 (1H, *d,* Arom), 9.98 (1H, *s*). ¹³C-NMR (CDCl₃) δ 24.2, 29.2, 71.1, 78.0, 123.5, 126.8, 127.8, 133.7, 135.5, 150.1, 170.6, 189.5. MS (CI) *m*/*z* 329 (M+1)⁺.

### 2-{[4,5-bis(nitrooxy)pentanoyl]oxy}benzoic acid (compound 6)

KMnO₄ (1.44 g, 9.14 mmol) was added to a stirred solution of 2-formylphenyl 4,5-bis(nitrooxy)pentanoate (2.0 g, 6.09 mmol) in acetone (60 mL), kept at 0 °C. The reaction was allowed to reach r.t and was completed after 1h (TLC detection eluent PE/EtOAc 70/30 v/v). Oxalic acid was added and the mixture was filtered and the filtrate was diluted with CH₂Cl₂ (50 mL). The organic layer was washed with H₂O (50 mL) and brine (50 mL) and then was dried with MgSO₄, filtered and concentrated under reduced pressure. The crude product was crystallized with PE/toluene 45/55 v/v to give the title compound as white solid (1.85 g).
Yield 89 %.
m.p. 92.5-93.0 °C (from PE/toluene 45/55 v/v).
TLC: Rf = 0.40 PE/EtOAc/HCOOH 70/30/0.1 v/v/v

¹H-NMR (CDCl₃) δ 2.13-2.25 (2H, *m*), 2.83 (2H, *t ,* J = 6.0 Hz), 4.54 (1H, *dd,* AMX like system), 4.84 (1H, *dd,* AMX like system), 5.50 (1H, *m*), 7.13 (1H, *d,* Arom), 7.40 (1H, *t,* Arom), 7.66 (1H, *t,* Arom), 8.14 (1H, *d,* Arom), 11.0 (1H, s *vvbr) .* ¹³C-NMR (CDCl₃) δ 24.6, 29.8, 71.4, 78.2, 122.0, 124.2, 126.9, 133.0, 135.6, 151.3, 169.9, 171.2. MS (CI) *m*/*z* 345 (M+1)⁺.

### Example 9

### 5,6-Dinitrooxyheptanoic acid

Iodine (1.98 g, 7.80 mmol) was added portion wise to a stirred solution of 6-heptenoic acid (1.06 mL, 7.80 mmol) and AgNO₃ (1.32 g, 7.80 mmol) in CH₃CN (20 mL) kept at -15 °C. At the end of the addition the stirring was continued for 30 min. Then AgNO₃ (2.64 g, 15.40 mmol) was added and the mixture was heated at 80 °C for 12 h. After cooling the mixture was filtered through Celite^{®}. The filtrate was concentrated under reduced pressure, dissolved in water (20 mL) and extracted with CH₂Cl₂ (3 x 20 mL). The combined organic layers were dried with MgSO₄, filtered and concentrated under reduced pressure to give the title compound as yellow oil (1.74 g).
Yield 88 %.
TLC: Rf = 0.35 PE/EtOAc/HCOOH 80/20/0.1 v/v/v

¹H-NMR (CDCl₃) δ 1.50-1.55 (2H, *m*), 1.67-1.80 (4H, *m*), 2.41 (2H, *t* , J = 7.1 Hz), 4.48 (1H, *dd,* AMX like system), 4.76 (1H, *dd,* AMX like system), 5.26-5.33 (1H, *m*). ¹³C-NMR (CDCl₃) δ 24.0, 24.3, 27.4, 33.5, 71.2, 78.6, 179.5. MS (CI) *m*/*z* 253 (M+1)⁺.

### 2-formylphenyl hept-6-enoate

SOCl₂ (350 µL, 4.75 mmol) and a few drops of dry DMF were added to a stirred solution of 5,6-dinitrooxyheptanoic acid (1.00 g, 3.96 mmol) in dry CH₂Cl₂ (10 mL) kept under N₂ at r.t. The stirring was continued for 2h at r.t. The solution of the acyl chloride so obtained was slowly added to a stirred solution of salycilic aldehyde (340 µL, 3.17 mmol) and dry Py (480 µL, 5.94 mmol) in dry CH₂Cl₂ (10 mL), kept under N2 at 0 °C. The reaction was allowed to reach r.t. and the stirring was continued for 18 h. The mixture was washed with HCl 2M (2 x 15 mL). The combined organic layers were dried with MgSO₄, filtered and concentrated under reduced pressure to give a crude product that was purified by flash chromatography (PE/EtOAc 85/15 v/v) to give the title compound as pale yellow oil (654 mg).
Yield 58 %.
TLC: Rf = 0.51 PE/EtOAc 80/20 v/v

¹H-NMR (CDCl₃) δ 1.55-1.67 (2H, m), 1.80-1.90 (4H, m), 2.71 (2H, t , J = 7.2 Hz), 4.50 (1H, dd, AMX like system), 4.78 (1H, dd, AMX like system), 5.29-5.37 (1H, m), 7.17 (1H, d, Arom), 7.43 (1H, t, Arom), 7.65 (1H, t, Arom), 7.87 (1H, d, Arom), 10.1 (1H, s). ¹³C-NMR (CDCl₃) δ 24.0, 24.3, 29.0, 33.4, 71.2, 79.0, 119.9, 123.5, 128.1, 132.2, 135.4, 151.0, 171.4, 189.0. MS (CI) m/z 357 (M+1)⁺.

### 2-{[6,7-bis(nitrooxy)heptanoyl]oxy}benzoic acid (compound 8)

KMnO₄ (0.43 g, 2.73 mmol) was added to a stirred solution of 2-formylphenyl 6,7-bis(nitrooxy)heptanoate (0.65 g, 1.82 mmol) in acetone (15 mL) kept at 0 °C. The mixture was allowed to reach r.t. and the reaction was completed after 2h (TLC detection, eluent PE/EtOAc 70/30 v/v). Oxalic acid was added and the mixture was filtered and the filtrate was diluted with CH₂Cl₂ (15 mL). The organic layer was washed with H₂O (15 mL) and brine (15 mL), then was dried with MgSO₄, filtered and concentrated under reduced pressure. The crude product was was purified by preparative HPLC (Lichrospher 250-25 C₁₈, CH₃CN/H₂O/TFA 60/40/0.1, flow 39 mL/min, λ 224 nm, injection 2 mL, solution 100 mg/mL) to give the title compound as white solid (349 mg).
Yield 89 %.
TLC: Rf = 0.40 PE/EtOAc/HCOOH 70/30/0.1 v/v/v

¹H-NMR (CDCl₃) δ 1.55-1.86 (4H, *m*), 2.67 (2H, *t,* J = 6.0 Hz), 4.47 (1H, *dd,* AMX like system), 4.74 (1H, *dd,* AMX like system), 5.30 (1H, *m*)*,* 7.13 (1H, *d,* Arom), 7.38 (1H, *t,* Arom), 7.65 (1H, *t,* Arom), 8.11 (1H, *d,* Arom), 8.49 (1H, s *br*). ¹³C-NMR (CDCl₃) δ 23.9, 24.3, 29.0, 33.6, 71.1, 78.9, 121.8, 124.0, 126.4, 132.5, 135.3, 151.1, 170.0, 172.2. MS (CI) *m*/*z* 373 (M+1)⁺.

### Example 10

### {4-[2,3-bis(nitrooxy)propoxy]phenyl}acetic acid

To a stirred solution of [4-(allyloxy)phenyl]acetic acid (2.00 g; 10.4 mmol; J. Chem. Soc. Perk. Trans. 1, 1985, 1629-1633) and AgNO₃ (1.77 g,10.4 mmol) in CH₃CN (60 mL) kept at -15 °C, iodine (2.64 g, 10.4 mmol) was added portion wise. At the end of the addition the mixture was stirred for 1h then AgNO₃ (3.54 g, 20,8 mmol) was added and the mixture was refluxed for 20 h. After cooling the mixture was filtered through Celite^{®}. The filtrate was concentrated under reduced pressure, dissolved in water (50 mL) and extracted with EtOAc (3 x 50 mL). The organic layers were dried with MgSO₄, filtered and concentrated under reduced pressure. The crude product was purified by flash chromatography (CH₂Cl₂/ EtOAc 85/15 v/v) to give the title compound as yellow oil. The compound was used immediately in the next synthetic step (2.12 g).
Yield 63 %.
TLC: Rf = 0.48 CH₂Cl₂/EtOAc/HCOOH 85/15/0.1 v/v/v

¹H-NMR (CDCl₃) δ 3.60 (2H, *s*), 4.22 (2H, *m*), 4.77 (1H, *dd,* AMX like system), 4.91 (1H, *dd,* AMX like system), 5.59 (1H, *m*), 6.86 (2H, *d,* Arom), 7.22 (2H, d).

### 2-[({4-[2,3-bis(nitrooxy)propoxy]phenyl}acetyl)oxy]benzoic acid (compound 9)

To a solution of {4-[2,3-bis(nitrooxy)propoxy]phenyl}acetic acid (2.00g; 6.33 mmol) in dry CH₂Cl₂ (20 mL), stirred under N₂ at r.t., were added few drops of dry DMF and SOCl₂ (555 µL; 7.60 mmol). The solution was stirred for 2 h at r.t..

To a solution of salycilic acid (612 mg; 4.43 mmol) in dry CH₂Cl₂ (30 mL), stirred at 0 °C under N₂, were added dry Py (768 µL; 9.50 mmol) and slowly the solution of acylchloride previously prepared. The mixture was allowed to reach r.t. and stirred for 2 hours. The mixture was washed twice with HCl 2M (40 mL). The organic layer was dried with MgSO₄, filtered and concentrated under reduced pressure. The crude product was partially purified by flash chromatography (PE/EtOAc 60/40 v/v), then was purified by preparative HPLC (Lichrospher 250-25 C₁₈, CH₃CN/H₂O/TFA 50/50/0.1, flow 39 mL/min, λ 224 nm, injection 3 mL, solution 65 mg/mL) to give the title compound as pale yellow solid (280 mg).
Yield 17 %.
mp 94.5-95.0 °C (from PE/toluene 50/50 v/v).
TLC: Rf = 0.31 PE/EtOAc/HCOOH 70/30/0.1 v/v/v

¹H-NMR (CDCl₃) δ 3.87 (2H, *s*), 4.16 (2H, *m*), 4.70 (1H, *dd,* AMX like system), 4.84 (1H, *dd,* AMX like system), 5.25 (1H, *m*), 6.84 (2H, *d,* Arom), 7.11 (1H, *d*, Arom), 7.28 (2H, *d, Arom*)*,* 7.37 (1H, *t*, Arom), 7.62 (1H, *t*, Arom), 8.09 (1H, *d,* Arom). ¹³C-NMR (CDCl₃) δ 40.2, 64.7, 68.9, 76.7, 114.7, 122.3, 123.9, 126.3, 126.8, 130.9, 132.4, 134.9, 151.1, 156.8, 170.0, 170.4. MS (CI) *m*/*z* 437 (M+1)⁺.

### Example 11

### [4-(3-nitrooxypropoxy)phenyl]acetic acid

A solution of [4-(3-bromopropoxy)phenyl]acetic acid (1.20 g, 4.39 mmol; Chem. Pharm. Bull. 1998, 46(1), 53-68) and AgNO₃ (1.50 g, 8.79 mmol) in CH₃CN (20 mL) was stirred at 70 °C for 8 h. The mixture was filtered and concentrated under reduced pressure. The residue was dissolved with CH₂Cl₂ (40 mL) and H₂O (40 mL). After separation organic layer was dried with MgSO₄, filtered and concentrated under reduced pressure. The crude product was dissolved with hot iPr₂O (10 mL) and riprecipitated with cold PE (40 mL) to give the title compound as white solid (690 mg).
Yield 62 %.
TLC: Rf = 0.33 PE/EtOAc 70/30 v/v

¹H-NMR (CDCl₃) δ 2.20 (2H, qi), 3.58 (2H, s), 4.05 (2H, t), 4.66 (2H, t), 6.85 (2H, d, Arom), 7.19 (2H, d, Arom). ¹³C-NMR (CDCl₃) δ 27.0, 40.1, 63.5, 70.0, 114.6, 125.8, 130.5, 157.7, 178.2. MS (CI) m/z 256 (M+1)⁺.

### 2-formylphenyl[4-(3-nitrooxypropoxy)phenyl]acetate

SOCl₂ (140 µL, 1.88 mmol) and a few drops of dry DMF were added to a stirred solution of [4-(3-nitrooxypropoxy)phenyl]acetic acid (0.40 g, 1.57 mmol) in dry CH₂Cl₂ (4 mL) kept under N₂ at r.t. The stirring was continued for 2h at r.t. The solution of the acyl chloride so obtained was slowly added to a stirred solution of salycilic aldehyde (135 µL, 1.26 mmol) and dry Py (190 µL, 2.35 mmol) in dry CH₂Cl₂ (5 mL), kept under N₂ at 0 °C. The reaction was allowed to reach r.t. and the stirring was continued for 26 h. The mixture was washed with HCl 2M (2 x 15 mL). The combined organic layers were dried with MgSO₄, filtered and concentrated under reduced pressure to give a crude product that was purified by flash chromatography (PE/EtOAc 90/10 v/v) to give the title compound as yellow oil (90 mg).
Yield 20 %.
TLC: Rf = 0.33 PE/EtOAc 80/20 v/v

¹H-NMR (CDCl₃) δ 2.22 (2H, qi) , 3. 91 (2H, *s*), 4.10 (2H, t), 4.68 (2H, t), 6.90 (2H, d, Arom), 7.15 (1H, d, Arom), 7.32 (2H, d, Arom), 7.38 (1H, t, Arom), 7.61 (1H, t, Arom), 7.86 (1H, d, Arom), 9.97 (1H, s). ¹³C-NMR (CDCl₃) δ 27.0, 40.3, 63.6, 70.0, 114.8, 123.4, 125.5, 126.5, 128.1, 130.6, 130.9, 135.3, 151.7, 157.9, 170.0, 188.6. MS (CI) m/z 360 (M+1)⁺.

### 2-[({4-[3-(nitrooxy)propoxy]phenyl}acetyl)oxy]benzoic acid (compound 10)

To a solution of 2-formylphenyl[4-(3-nitrooxypropoxy)phenyl]acetate (0.09 g, 0.24 mmol) in CH₃CN (640 µL) kept at 0 °C were added a solution of KH₂PO₄ (0.03 g) in H₂O (425 µL) and H₂O₂ 35 % (25 µL, 0.26 mmol) and dropwise a solution of NaClO₂ (0.04 g, 0.33 mmol) in H₂O (425 µL). After 1h the reaction was completed. Na₂SO₃ was added to destroy the excess of H₂O₂. After acidification with HCl 6M the mixture was diluted with H₂O (10 mL) and extracted twice with CH₂Cl₂ (15 mL). The organic layer was dried with MgSO₄, filtered and concentrated under reduced pressure to give a crude product that was purified by flash chromatography (CH₃CN/H₂O/TFA 50/50/0.1) to give the title compound as white solid (48 mg).
Yield 53 %.
mp 81.1-83.0 °C (from toluene).
TLC: Rf = 0.30 PE/EtOAc/HCOOH 70/30/0.1 v/v/v

### 2-[({4-[3-(nitrooxy)propoxy]phenyl}acetyl)oxy]benzoic acid (compound 10)

To a solution of [4-(3-nitrooxypropoxy)phenyl]acetic acid (0.26 g; 1.00 mmol) in dry CH₂Cl₂ (5 mL), stirred under N₂ at r.t., were added few drops of dry DMF and SOCl₂ (87 µL; 1.20 mmol). The solution was stirred for 3.5 h at r.t.. To a solution of salycilic acid (97 mg; 0.70 mmol) in dry CH₂Cl₂ (5 mL), stirred at 0 °C under N₂, were added dry Py (120 µL; 1.50 mmol) and slowly the solution of acylchloride previously prepared. The mixture was allowed to reach r.t. and stirred for 18 hours. The mixture was washed twice with HCl 2M (10 mL). The organic layer was dried with MgSO₄, filtered and concentrated under reduced pressure. The crude product was purified by flash chromatography (CH₃CN/H₂O/TFA 40/60/0.1, RP18) to give the title compound as white solid (129 mg).
Yield 49 %.
mp 81.1-83.0 °C (from toluene).
TLC: Rf = 0.30 PE/EtOAc/HCOOH 70/30/0.1 v/v/v

¹H-NMR (CDCl₃) δ 2.14 (2H, m), 3.87 (2H, s), 4.00 (2H, t), 4.62 (2H, t), 6.84 (2H, d, Arom), 7.10 (1H, d, Arom), 7.27 (2H, d, Arom), 7.34 (1H, t, Arom), 7.60 (1H, t, Arom), 8.11 (1H, d, Arom), 9.72 (1H, s broad). ¹³C-NMR (CDCl₃) δ 26.9, 40.2, 63.5, 70.0, 114.6, 122.3, 123.9, 125.8, 126.2, 130.8, 132.4, 134.8, 151.2, 157.7, 169.9, 170.5. MS (CI) m/z 376 (M+1)⁺.

### Example 12

### 3-{[5-oxido-4-phenyl-1,2,5-oxadiazol-3-yl]oxy}propanoic acid

A solution of Jones reagent 2.5 M (21.0 mL, 52.9 mmol) was added to a stirred solution of 3-{[5-oxido-4-phenyl-1,2,5-oxadiazol-3-yl]oxy}propan-1-ol (5.00 g, 21.2 mmol; Lolli et al. J. Med. Chem. 2001, 44, 3463) in acetone (150 mL), cooled at 0 °C. The mixture was allowed to reach r.t. and stirred for 18h. iPrOH (15 mL) was added and the mixture was concentrated under reduced pressure. The residue was dissolved with EtOAc (50 mL) and extracted with a saturated solution of NaHCO₃ (50 mL). The aqueous layers were acidified with HCl 6M and extracted twice with EtOAc (3 x 100 mL). The combined organic layers were dried with MgSO₄, filtered and concentrated under reduced pressure to give the title compound as white solid (3.1 g).
Yield 59 %.
m.p. 136.5-137.0 °C (from toluene).
TLC: Rf = 0.31 CH₂Cl₂/EtOAc 95/5 v/v

¹H-NMR (DMSO-d₆) δ 3.01 (2H, *t*, J = 6.0 Hz) , 4.78 (2H, *t*, J = 6.0 Hz), 7.41-7.50 (3H, *m,* Arom), 8.05-8.07 (2H, *m,* Arom), 11.41 (1H*, s vvbr*). ¹³C-NMR (DMSO-d₆) δ 33.3, 66.7, 107.3, 121.9, 125.7, 128.8, 129.3, 161.9, 171.6 . MS (CI) *m*/*z* 251 (M+1)⁺.

### 2-[(3-{[5-oxido-4-phenyl-1,2,5-oxadiazol-3-yl]oxy}propanoyl)oxy]benzoic acid (compound 12)

SOCl₂ (175 µL, 2.40 mmol) and a few drops of dry DMF were added to a solution of 3-{[5-oxido-4-phenyl-1,2,5-oxadiazol-3.yl]oxy}propanoic acid (0.50 g, 2.00 mmol) in dry CH₂Cl₂ (5 mL), stirred under N₂ at r.t. The stirring was continued for 18 h at r.t. The solution of the acyl chloride so obtained was slowly added to a stirred solution of salycilic acid (0.20 g, 1.40 mmol) and dry Py (240 µL, 3.00 mmol) in dry CH₂Cl₂ (5 mL) kept under N₂ at 0 °C. The mixture was allowed to reach r.t. and then stirred for 5h. The mixture was washed twice with HCl 2M (15 mL). The combined organic layers were dried with MgSO₄, filtered and concentrated under reduced pressure. The crude product was partially purified by preparative HPLC (Lichrospher 250-25 C₁₈, CH₃CN/H₂O/TFA 50/50/0.1, flow 39 mL/min, λ 224 nm, injection 4 mL, solution 50 mg/mL) to give the title compound as white solid (310 mg).
Yield 61 %.
mp 152.0-153.9°C (from toluene).
TLC: Rf = 0.34 PE/EtOAc/HCOOH 60/40/0.1 v/v/v

¹H-NMR (DMSO-d₆) δ 3.27 (2H, *t,* J = 6.0 Hz), 4.80 (2H, *t*, J = 6.0 Hz), 7.14-8.07 (9H, *m,* Arom), 13.16 (1H, *s br*). ¹³C-NMR (DMSO-d₆) δ 33.9, 66.5, 107.9, 122.3, 124.1, 124.2, 126.6, 126.7, 129.3, 131.1, 131.9, 134.3, 150.3, 162.4, 165.8, 169.4. MS (CI) *m*/*z* 371 (M+1)⁺.

### Hydrolysis experiments

### Hydrolysis in acidic medium (pH 1).

A solution of each compound (10 mM) in acetonitrile was added to a HCl 0.1M preheated at 37°C, the final concentration of the compound was 250 µM. Resulting solution was maintained at 37 ±0.5°C and at appropriate time intervals a 20 µL aliquote of reaction solution was analysed by RP-HPLC.

### Hydrolysis in human serum.

A solution of each compound (10 mM) in acetonitrile was added to human serum (Sigma) preheated at 37°C, the final concentration of the compound was 250 µM. Resulting solution was incubated at 37 ±0.5°C and at appropriate time intervals 500 µL of reaction mixture was withdrawn and added to 750 µL of acetonitrile containing 0.1% trifluoroacetic acid in order to deproteinize the serum. Sample was sonicated, vortexed and then centrifuged for 10' at 2150 g, The clear supernatant was filtered by 0.45 µm PTFE filters (Alltech) and analysed by RP-HPLC.

The reverse-phase HPLC procedure allowed separation and quantitation of remaining salicylic ester and of salicylic acid.

HPLC analyses were performed with a HP 1100 chromatograph system (Agilent Technologies, Palo Alto, CA, USA) equipped with a quaternary pump (model G1311A), a membrane degasser (G1379A), a diode-array detector (DAD) (model G1315B) integrated in the HP1100 system. Data analysis was done using a HP ChemStation system (Agilent Technologies). The analytical column was a Nucleosil 100-5C18 Nautilus (250 x 4.6 mm, 5 µm particle size) (Macherey-Nagel). The mobile phase consisting of acetonitrile/water (55/45) with 0.1% trifluoroacetic acid and the flow-rate was 1.2 mL/min. The injection volume was 20 µL (Rheodyne, Cotati, CA). The column effluent was monitored at 226 nm (for salicylic esters) and 240 nm (for salicylic acid) referenced against a 600 nm wavelength. Quantitation was done by comparison of peak areas with standards chromatographed under the same conditions.

The hydrolysis of all esters followed first-order kinetics; the observed pseudo-first-order rate constants (k_{obs}) for the hydrolysis were calculated from the slopes of linear plots of the natural logarithm of percent remaining salicylic ester against time and the corresponding half-lives (t_{1/2}) were obtained from: ${t}_{1 / 2} = 0.693 / {k}_{obs}$

Results are reported in Table 1.

**Table 1**

| Compound | Uman Serum stability (t_{1/2} h) | pH 1 stability (% after 3h) |
|---|---|---|
| **Acetylsalicylic acid** | 1.37 | > 90 % |
| **Compound (1)** | 0.26 | > 90 % |
| **Compound (7)** | 4.87 | > 85 % |
| **Compound (11)** | 0.44 | > 90 % |
| **Compound (6)** | 4.08 | > 85 % |

### Anti-inflammatory activity

Paw edema was induced in conscious rats by subplantar injection of carrageenan (0.1 ml of 1% carrageenan suspension in carboxymethylcellulose 1%). Immediately after carrageenan injection, compounds or vehicle (CMC, 1%) were administered intragastrically to different groups of rats in a volume of 10 ml/kg. Paw volume was measured with a plethysmometer (Basile, Comerio, Italy) immediately before carrageenan injection and 3 hours afterwards. Paw edema was determined in each rat by subtracting the initial volume displacement (pre-drug) from the subsequent post-carrageenan measurement. Edema was expressed as the percent increase in paw volume relative to the preinjection value for each animal. The results obtained are presented in the Table 2 as mean ± SEM. Statistical analysis was performed with ANOVA followed by Newman Keuls test.

### Gastrolesive activity

Male Wistar rats, weighing 180-200 (Harlan, S. Pietro al Natisone, Italy) were individually housed in hanging stainless-steel cages with grid floors, at constant room temperature (25±1 °C) and humidity (60±5%), with an artificial 12:12 h light/dark cycle. They were deprived of food but not of water 24 h before the experiments. Groups of rats (n = 8-10) were given aspirin (Acetylsalicylic acid) 120 mg/kg by intragastric route or equimolar doses of the compounds under study. Rats were sacrificed 3 hours after the administration of the compounds. Immediately after the sacrifice, the stomachs were removed, opened along the lesser curvature and examined for the assessment of mucosal lesions, the stomachs were layed on a flat surface under a stereomicroscope. The glandular mucosa was examined and each individual hemorrhagic lesion was measured along its greatest length (<1 mm: rating=1; 1-2 mm: rating=2; >2 mm: rating according to their greatest length). The lengths of the lesions were summed to give an overall total, designated as the lesion index, for each stomach. The results obtained are presented in the Table 2 as mean ± SEM. Statistical analysis was performed with ANOVA followed by Newman Keuls test.

**Table 2**

| Compound | Anti inflammatory activity | Gastrotoxicity |
|---|---|---|
| | Paw edema (% increase) | Lesion index (mm) |
| **Control** | 58.80 ± 4.69 | |
| **Acetylsalicylic acid** | 32.95 ± 3.19** | 44.75 ± 4.66 |
| **Compound (7)** | 26.19 ± 3.58** | 0.85 ± 0.64# |
| **Compound (11)** | 34.88 ± 4.69* | 0.50 ± 0.31# |
| **Compound (6)** | 29.95 ± 7.30** | 0.50 ± 0.22# |
| **Propanoylsalicylic acid** | 48.13 ± 6.54 | 52.00 ± 11.78 |

| | | |
|---|---|---|
| * P<0.005 vs control; ** P<0.001 vs control # P<0.001 vs Acetylsalicylic acid. Values are mean ± SEM from 6-9 rats per group. Paw edema volume and gastric lesions were determined 3 hours after treatments. | | |

### Inhibitory effects on cyclooxigenase type I

The extent and type of inhibitory effects of this class of compounds on cyclooxygenase type-1 (COX1) activity was estimated in resting RAW 264.7 macrophages. Arachidonic acid (1µM) was used as the substrate of the enzyme and the extent of PGE2 released in the incubating buffer was used as an index of enzyme activity.

The type of inhibition (irreversible vs reversible) was determined in resting conditions by exposing the cells to the test compounds for 30 min followed by the application of the substrate either directly or after extensive washing and then incubated for additional 15 min. The presence of residual activity (difference less than 5% vs that recorded without washing) was taken as index of the irreversible nature of the inhibitory effects of the compounds.

### Inhibitory effects on cyclooxigenase type II

The inhibitory activity on COX2 was, instead, analyzed following the induction of this protein with 1 µg/mL of the bacterial endotoxin, lipopolysaccharide (LPS) and 10 ng/mL of interferon-γ (IFNγ) that were applied for 16 h to RAW 264.7 macrophages previously treated with 100µM of the irreversible blocker, aspirin (ASA). Arachidonic acid served as substrate for the enzyme and the extent of PGE2 formation taken as index of COX2 activity.
Compounds belonging to this new class of nitro-acyl derivatives of salicylic acid resulted unexpectedly more potent than Acetylsalicylic acid (ASA) yet maintained their activity in an irreversible fashion (Table 3).

**Table 3**

| Inhibitory effects of various NO-ASA derivatives on cyclooxygenase type-1 (COX1) and type-2 (COX2) | | | | |
|---|---|---|---|---|
| **Compound** | **Concentration (µM)** | **COX1 inhibition %** | **COX2 inhibition %** | **Type of inhibition "Irreversibile"** |
| **ASA** | 30 | 53 | 20 | YES |
| | 10 | 27 | 5 | |
| | 1 | NE | 2 | |
| **Compound (2)** | 30 | 77 | 44 | YES |
| | 10 | 62 | 15 | |
| | 1 | 21 | 6 | |
| **Compound (3)** | 30 | 77 | 43 | YES |
| | 10 | 66 | 27 | |
| | 1 | 11 | 5 | |

### Inhibitory effects on platelet aggregation

Platelets are prominent components of the thrombi. Platelet aggregation was measured in PRP using a Chrono-Log platelet aggregometer. The platelets were stimulated by arachidonic acidic (1 mM). The inhibitory activity of Acetylsalicylic acid (ASA) and the compound of the invention was tested by adding the compounds to PRP 5 min before the stimulus of arachidonic acidic. The compound belonging to this new class of nitro-acyl derivatives of salicylic acid resulted unexpectedly more potent than Acetylsalicylic acid (ASA) to inhibit platelet aggregation (Table 4).

**Table 4**

| **Compound** | **Concentration (µM)** | **Platelet aggregation inhibition %** |
|---|---|---|
| **ASA** | 30 | 53 |
| | 10 | 32 |
| **Compound (2)** | 30 | 96 |
| | 10 | 66 |

## Claims

1. A compound of general formula (I) and pharmaceutically acceptable salts or stereoisomers thereof: 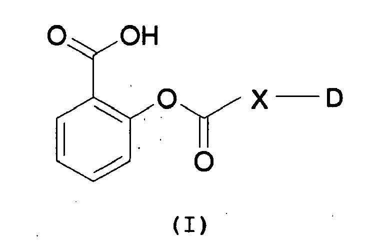 wherein:
D is -ONO₂ or 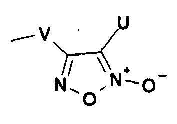 wherein V is -CH₂-, -O-, -S- or - NH-; U is C₁-C₁₀ alkyl, optionally substituted with -OH or -NH₂, aryl, C₁-C₁₀ alkoxy, aryloxy, C₁-C₁₀ thioalkyl, thioaryl, halogen, di-C₁-C₁₀(alkylamino), diarylamino, arylC₁-C₁₀ (alkylamino), C₁-C₁₀ (alkylsulphoxy), arylsulphoxy, C₁-C₁₀ (alkylsulphone), arylsulphone, -CN, -NO₂, -NHCOR₀, -COR₀, -COOR₀, -CON(R₀) (R₁), wherein R₀ and R₁ are the same or different, and are H, alkyl or aryl;
X is a bivalent radical having the following meanings:
a) straight or branched C₁-C₂₀ alkylene, optionally substituted with one or more of the substituents selected from the group consisting of halogen atom, -OH, -COOH, -ONO₂ or T, wherein T is -OC (O) (C₁-C₁₀ alkyl) -ONO₂ or -O (C₁-C₁₀ alkyl) -ONO₂;
b) a C₅-C₇ cycloalkylene group optionally substituted with linear or branched C₁-C₁₀ alkyl group;
c) 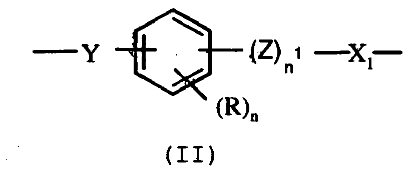 wherein:
Y is straight or branched C₁-C₂₀ alkylene, or -CH=CH-(CH₂)ₙ²-wherein n² is an integer from 0 to 10;
R is H, C₁-C₅ alkyl, -COOH, or -OR' wherein R' is H or a C₁-C₃ alkyl group;
Z is O, -C(O)O- or -OC(O)-;
n is 0 or 1;
n¹ is 0 or 1;
X₁ is a straight or branched C₁-C₂₀ alkylene, optionally substituted with one or more of the substituents selected from the group consisting of halogen atoms, -OH, -COOH, -ONO₂ or X₁ is a group of formula (III) 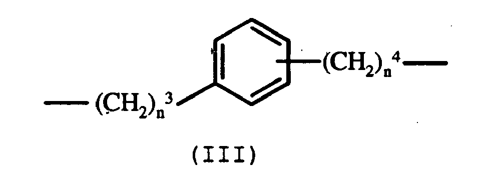
wherein:
n³ is an integer from 0 to 5;
n⁴ is an integer from 1 to 5;
wherein, the group D of formula (I) is bound to the X₁ group of formula (II), and to the -(CH₂)ₙ⁴- group of formula (III);
d) 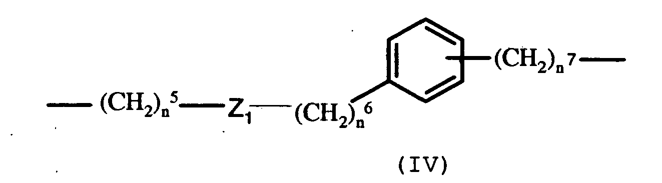 wherein n⁵ is an integer from 1 to 20;
Z₁ is -C(O)O- or -OC(O) -:
n⁶ is an integer from 0 to 20;
n⁷ is an integer from 1 to 20;
wherein the group D of formula I) is bound to -(CH₂)ₙ⁷-group;
e) 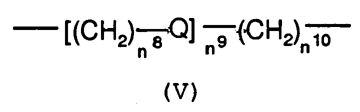 wherein
Q is O or S;
n⁸ is an integer from 1 to 6;
n⁹ is an integer from 1 to 10;
n¹⁰ is an integer from 1 to 10;
f) 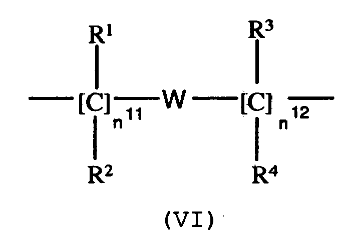 wherein:
n¹¹ is an integer from 0 to 10;
n¹² is an integer from 1 to 10;
R¹, R², R³, R⁴ are the same or different, and they are H or
straight or branched C₁-C₄ alkyl;
wherein the D group of formula (I) is linked to 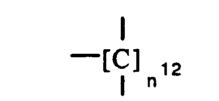 W is an heterocyclic saturated, unsaturated or aromatic 5 or 6 members ring, containing one or more heteroatoms selected from nitrogen, oxygen, sulfur, and is selected from

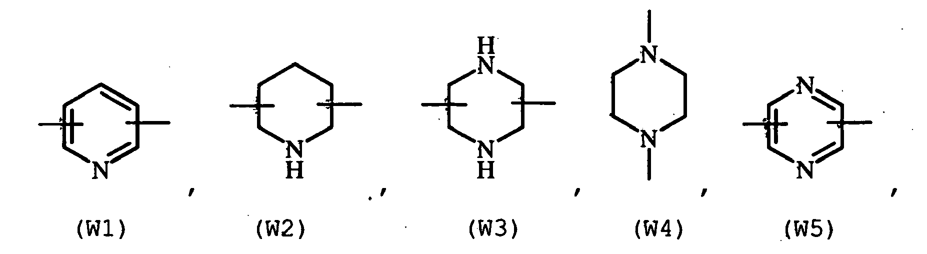

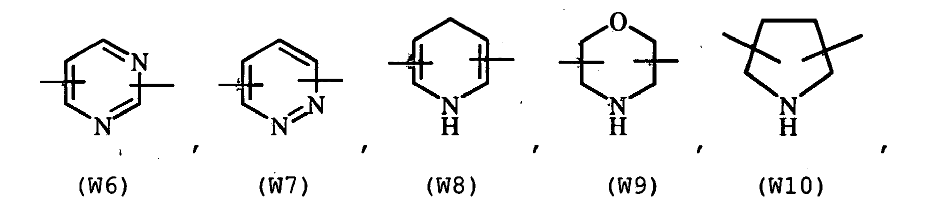

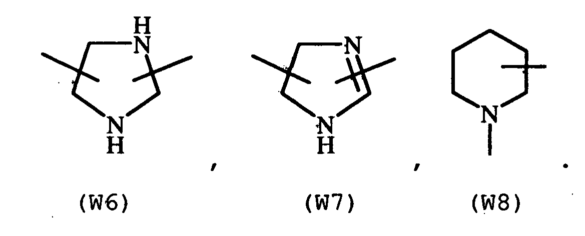

2. A compound of formula (I) according to claim 1, wherein:
X is:
a) straight or branched C₁-C₁₀ alkylene, optionally substituted with one or more of the substituents selected from the group consisting of halogen atom,-OH,-COOH,-ONO₂ or T, wherein T is -OC(O)(C₁-C₁₀ alkyl) -ONO₂ or -O(C₁-C₁₀ alkyl)-ONO₂;
c) 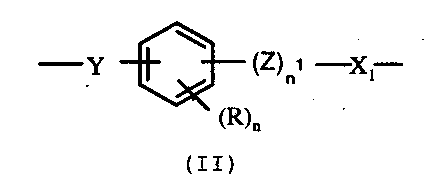 wherein:
Y is straight or branched C₁-C₆ alkylene, or -CH=CH-(CH₂)ₙ²-wherein n² is 0 or 1;
R is H, -CH₃, -COOH, or -OR' wherein R' is H or -CH₃;
Z is O, -OC(O)- ;
n is 0 or 1
n¹ is 0 or 1;
X₁ is a straight or branched C₁-C₁₀ alkylene, optionally substituted with one or more of the substituents selected from the group consisting of halogen atoms, -OH, -COOH, -ONO₂ or X₁ is a group of formula (III) : 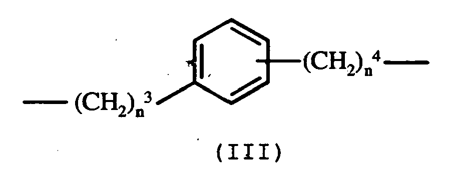 wherein:
n³ is 0 or 1;
n⁴ is 1;
wherein, the group D of formula (I) is bound to the X₁ group of formula (II), and to the - (CH₂)ₙ⁴- group of formula (III);
d) 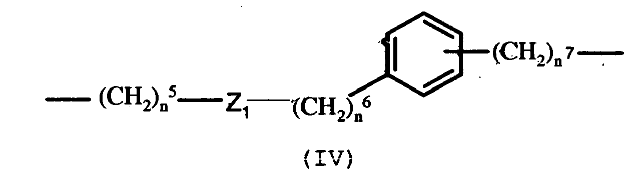 wherein n⁵ is an integer from 1 to 10;
Z₁ is -C(O)O- or -OC(O)-;
n⁶ is an integer from 0 to 10;
n⁷ is an integer from 1 to 10;
wherein the group D of formula I) is bound to - (CH₂)ₙ⁷-group;
e) 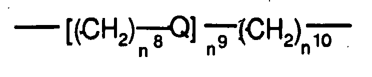 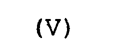 wherein
Q is -O- or -S-;
n⁸ is an integer from 1 to 4;
n⁹ is an integer from 1 to 6;
n¹⁰ is an integer from 1 to 6;
f) 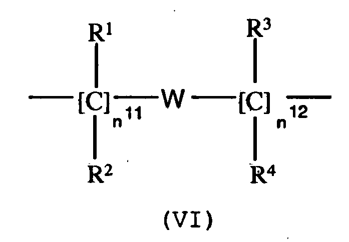 wherein:
n¹¹ is an integer from 0 to 4;
n¹² is an integer from 1 to 4;
R¹, R², R³, R⁴ are H;
wherein the D group of formula (I) is linked to 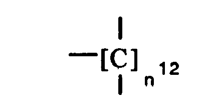 W is an heterocyclic ring selected from:

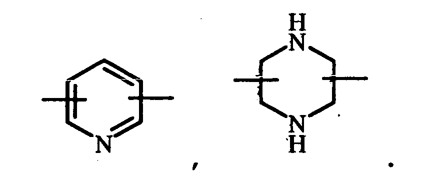

3. A compound of formula (I) according to claim 1, wherein:
X is:
a) a straight or branched C₁-C₁₀ alkylene, optionally substituted with one or more -ONO₂ groups;
c) 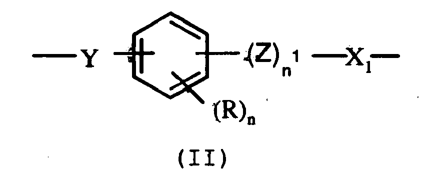 wherein:
Y is straight or branched C₁-C₆ alkylene, or -CH=CH-(CH₂)ₙ²-wherein n² is 0 or 1;
R is H or -OR' wherein R' is CH₃;
Z is O or -OC(O)-;
n is 0 or 1;
n¹ is 0 or 1;
X₁ is a straight or branched C₁-C₆ alkylene, optionally substituted with one or more -ONO₂ groups or X₁ is a group of formula (III): 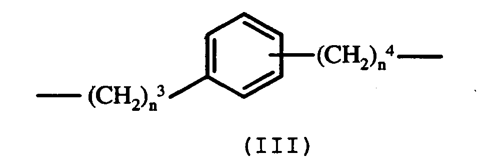
d) 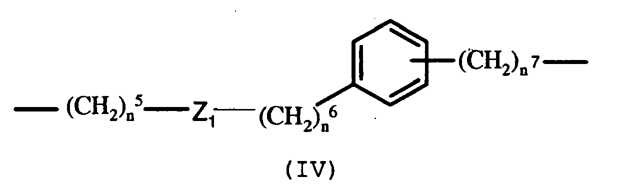 wherein n⁵ is an integer from 1 to 5;
Z₁ is -C(O)O- or -OC(O)-;
n⁶ is an integer from 0 to 5;
n⁷ is an integer from 1 to 5;
wherein the group D of formula I) is bound to -(CH₂)ₙ⁷-group;
e) 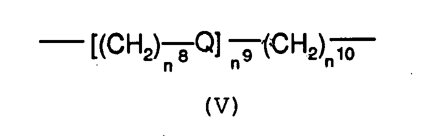 wherein
Q is O;
n⁸ is 1 or 2;
n⁹ is an integer from 1 to 4;
n¹⁰ is an integer from 1 to 2.
wherein:
n³ is 0 or 1;
n⁴ is 1;
wherein, the group D of formula (I) is bound to the X₁ group of formula (II), and to the -(CH₂)ₙ⁴- group of formula (III) ;

4. A compound of formula(I) according to claim 1 selected from the group:

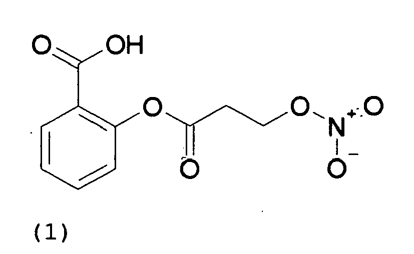

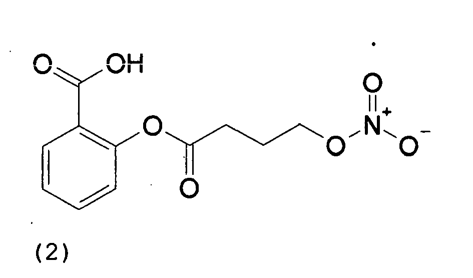

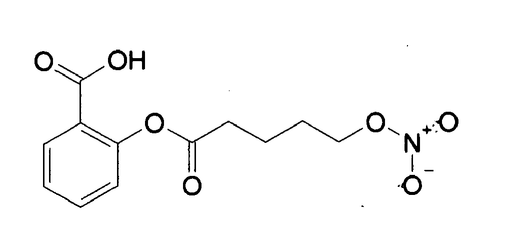

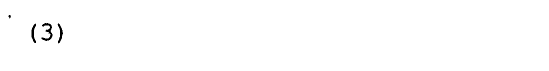

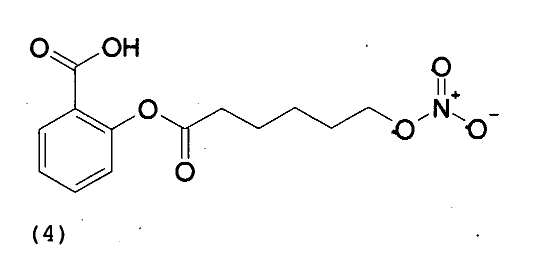

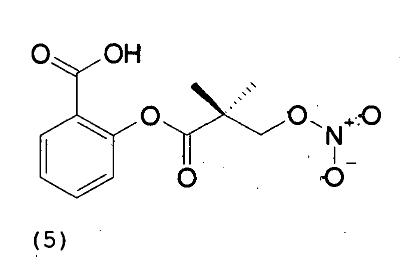

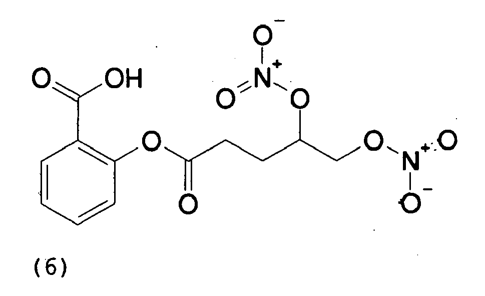

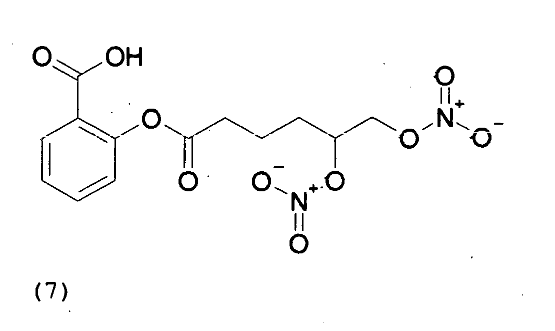

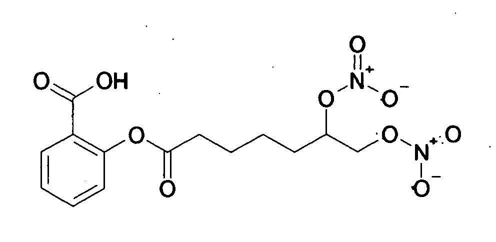

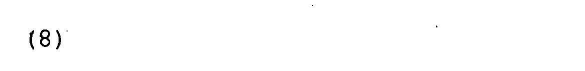

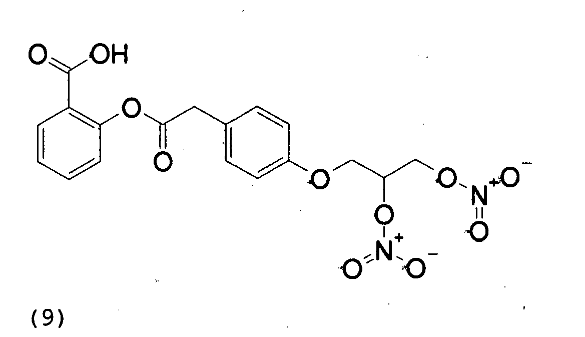

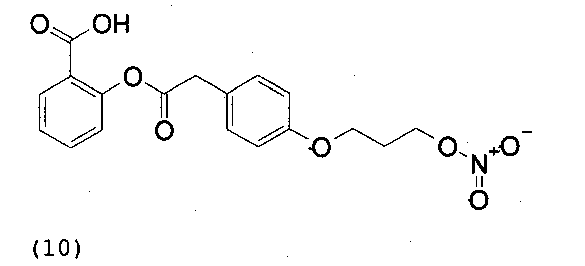

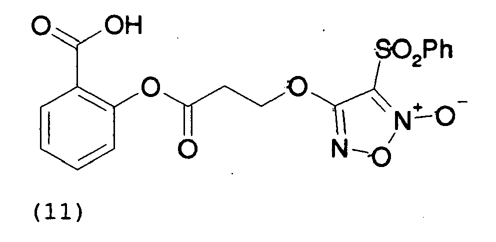

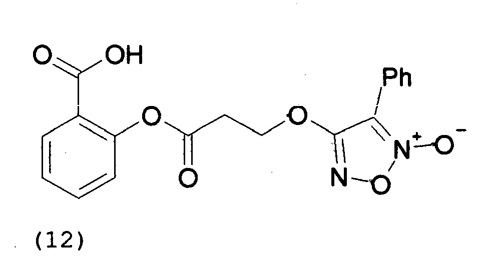

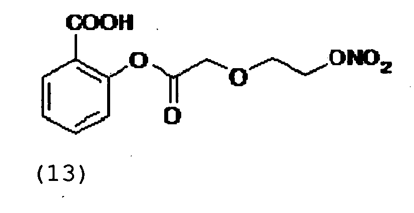

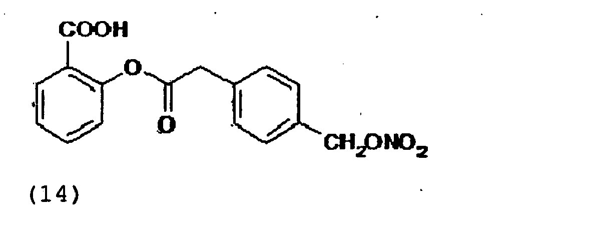

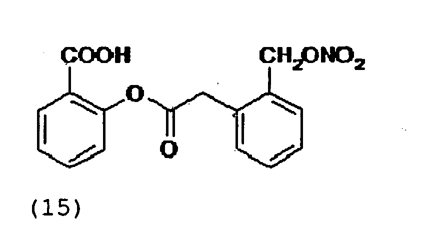

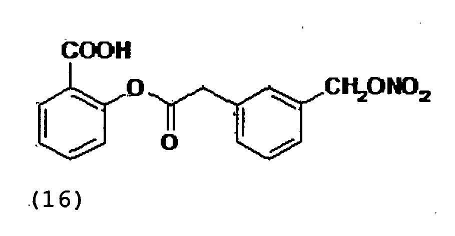

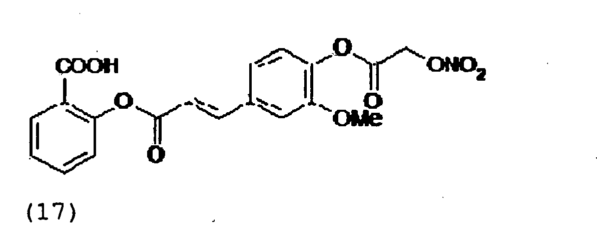

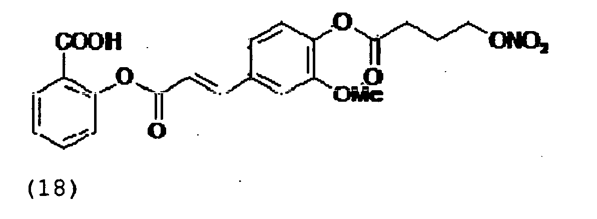

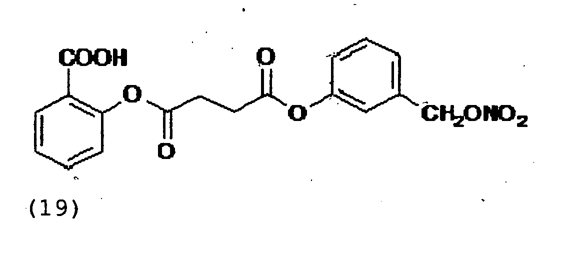

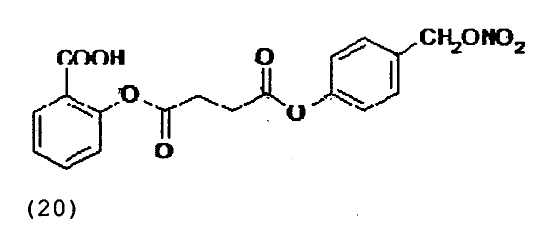

5. A process for preparing a compound of formula (I) according to claim 1, comprising the oxidation of a compound of formula (VIII) : 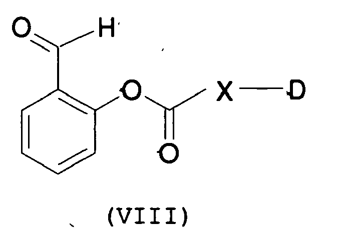 wherein X and D are as defined in claim 1.

6. A compound of general formula (I) according to claims 1-4 for use as a medicament.

7. Use of a compound according to claims 1-4 for the preparation of an medicament having anti-inflammatory, antithrombotic and antiplatelet activity.

8. Use of a compound according to claims 1-4 for the preparation of an medicament for treating inflammation, pain, fever and cardiovascular diseases.

9. Use of a compound according to claims 1-4 for the preparation of an medicament for preventing or treating cancer diseases.

10. Use of a compound according to claim 9 for the preparation of an medicament for treating colon cancer, bladder cancer, prostate cancer.

11. A pharmaceutical composition comprising a pharmaceutically acceptable carrier and a pharmaceutically effective amount of a compound of general formula (I) and/or a salt or stereoisomer thereof as defined in claims 1-4.

## Patentansprüche

1. Verbindung gemäß der allgemeinen Formel (I) und pharmazeutische akzeptable Salze oder Stereoisomere davon: 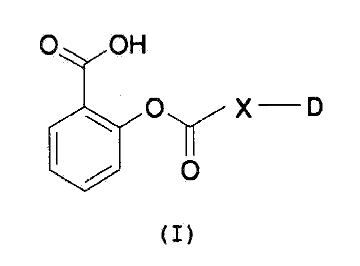 worin:
D -ONO₂ oder 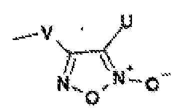 ist, worin V -CH₂-, -O-, -S- oder -NH- ist; U C₁-C₁₀-Alkyl, ggf. substituiert mit -OH oder -NH₂, Aryl, C₁-C₁₀-Alkoxy, Aryloxy, C₁-C₁₀-Thioalkyl, Thioaryl, Halogen, Di-C₁-C₁₀-(alkylamino), Diarylamino, Aryl-C₁-C₁₀-(alkylamino), C₁-C₁₀-(Alkylsulfoxy), Arylsulfoxy, C₁-C₁₀-(Alkylsulfon), Arylsulfon, -CN, -NO₂, -NHCOR₀, -COR₀, -COOR₀, -CON(R₀)(R₁), worin R₀ und R₁ gleich oder verschieden sind und H Alkyl oder Aryl sind;
X ein zweiwertiger Rest mit den folgenden Bedeutungen ist:
a) gradkettiges oder verzweigtes C₁-C₂₀-Alkylen, ggf. subsituiert mit einem oder mehreren Substituenten, ausgewählt aus der Gruppe, bestehend aus Halogenatom, -OH, -COOH, -ONO₂ oder T, worin T -OC(O)(C₁-C₁₀-Alkyl)-ONO₂ oder -O(C₁-C₁₀-Alkyl)-ONO₂ ist;
b) eine C₅-C₇-Cycloalkylengruppe, ggf. substituiert mit einer geradkettigen oder verzweigten C₁-C₁₀-Alkylgruppe;
c) 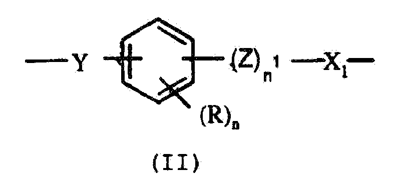 worin:
Y ein geradkettiges oder verzweigtes C₁-C₂₀-Alkylen oder -CH=CH-(CH₂)ₙ²- ist, worin n² eine ganze Zahl von 0 bis 10 ist;
R H, C₁-C₅-Alkyl, -COOH oder -OR' ist, worin R' H oder eine C₁-C₃-Alkylgruppe ist;
Z O, -C(O)O- oder -OC(O)- ist;
n 0 oder 1 ist;
n¹ 0 oder 1 ist;
X₁ ein geradkettiges oder verzweigtes C₁-C₂₀-Alkylen ist, das ggf. mit einer oder mehreren Substituenten substituiert ist, die aus der Gruppe ausgewählt werden, die aus Halogenatomen, -OH, -COOH, -ONO₂ besteht oder X₁ ist eine Gruppe der Formel (III) 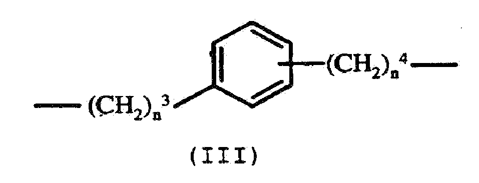
worin:
n³ eine ganze Zahl von 0 bis 5 ist;
n⁴ eine ganze Zahl von 1 bis 5 ist;
worin die Gruppe D der Formel (I) an die X₁-Gruppe der Formel (II) und an die -(CH₂)ₙ⁴-Gruppe der Formel (III) gebunden ist;
d) 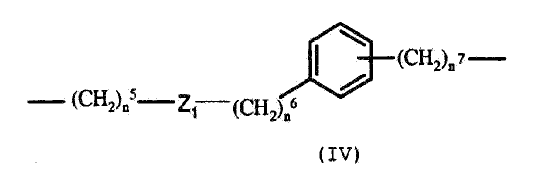 worin n⁵ eine ganze Zahl von 1 bis 20 ist;
Z₁ -C(O)O- oder -OC(O)- ist;
n⁶ eine ganze Zahl von 0 bis 20 ist;
n⁷ eine ganze Zahl von 1 bis 20 ist;
worin die Gruppe D der Formel I) an die -(CH₂)ₙ⁷-Gruppe gebunden ist;
e) 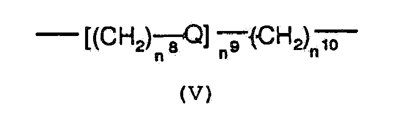 worin
X O oder S ist;
n⁸ eine ganze Zahl von 1 bis 6 ist;
n⁹ eine ganze Zahl von 1 bis 10 ist;
n¹⁰ eine ganze Zahl von 1 bis 10 ist;
f) 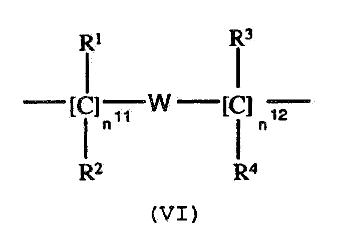 worin:
n¹¹ eine ganze Zahl von 0 bis 10 ist;
n¹² eine ganze Zahl von 1 bis 10 ist;
R¹, R², R³, R⁴ gleich oder verschieden sind und H oder ein geradkettiges oder verzweigtes C₁-C₄-Alkyl sind;
worin die D-Gruppe der Formel (I) an 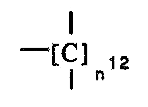 gebunden ist,
W ein heterocyclischer gesättigter, ungesättigter oder aromatischer 5- oder 6-gliedriger Ring ist, der ein oder mehrere Heteroatome, ausgewählt aus Stickstoff, Sauerstoff, Schwefel, enthält und ausgewählt wird aus

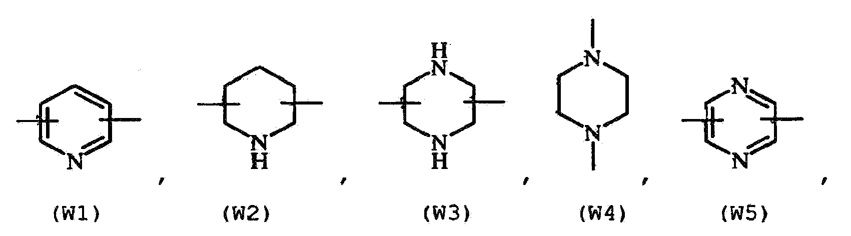

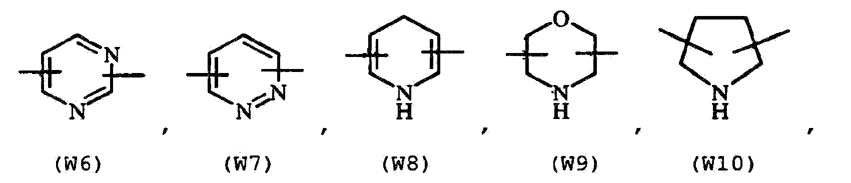

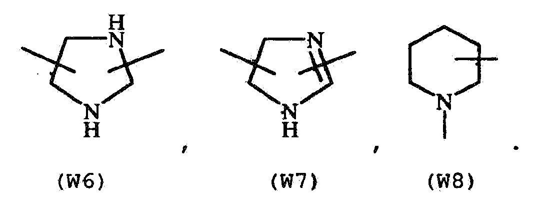

2. Verbindung der Formel (I) gemäß Anspruch 1, worin:
X folgendes ist:
a) geradkettiges oder verzweigtes C₁-C₁₀-Alkylen, ggf. substituiert mit einem oder mehreren Substituenten, ausgewählt aus der Gruppe, bestehend aus Halogenatom, -OH, -COOH, -ONO₂ oder T, worin T -OC(O)(C₁-C₁₀-Alkyl)-ONO₂ oder -O(C₁-C₁₀-Alkyl)-ONO₂;
c) 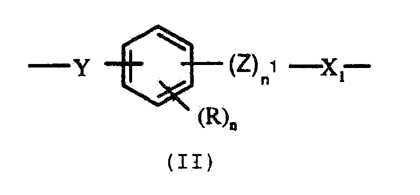 worin:
Y ein geradkettiges oder verzweigtes C₁-C₆-Alkylen oder -CH=CH-(CH₂)ₙ²- ist, worin n² 0 oder 1 ist;
R H, -CH₃, -COOH oder -OR' ist, worin R' H oder -CH₃ ist;
Z O, -OC(O)- ist;
n 0 oder 1 ist;
n¹ 0 oder 1 ist;
X₁ ein geradkettiges oder verzweigtes C₁-C₁₀-Alkylen ist, das ggf. mit einem oder mehreren Substituenten substituiert ist, die aus der Gruppe ausgewählt werden, die aus Halogenatomen, -OH, -COOH, -ONO₂ besteht, oder X₁ ist eine Gruppe der Formel (III): 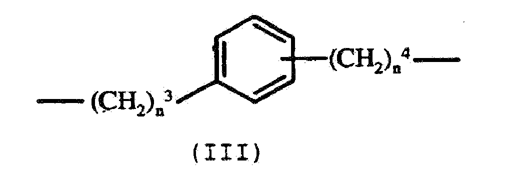
worin:
n³ 0 oder 1 ist;
n⁴ 1 ist;
worin die Gruppe D der Formel (I) an die X₁-Gruppe der Formel (II) und an die -(CH₂)ₙ⁴-Gruppe der Formel (III) gebunden ist;
d) 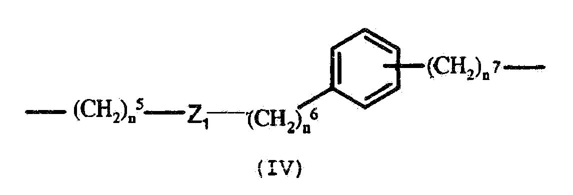
worin n⁵ eine ganze Zahl von 1 bis 10 ist;
Z₁ -C(O)O- oder -OC(O)- ist;
n⁶ eine ganze Zahl von 0 bis 10 ist;
n⁷ eine ganze Zahl von 1 bis 10 ist;
worin die Gruppe D der Formel (I) an die -(CH₂)ₙ⁷-Gruppe gebunden ist;
e) 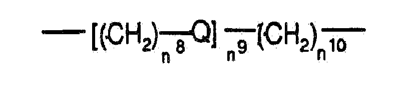 worin
Q -O- oder -S- ist;
n⁸ eine ganze Zahl von 1 bis 4 ist;
n⁹ eine ganze Zahl von 1 bis 6 ist;
n¹⁰ eine ganze Zahl von 1 bis 6 ist;
f) 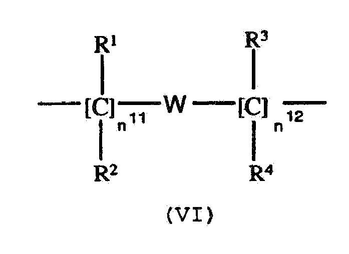 worin:
n¹¹ eine ganze Zahl von 0 bis 4 ist;
n¹² eine ganze Zahl von 1 bis 4 ist;
R¹, R², R³, R⁴ H sind
worin die D-Gruppe der Formel (I) an 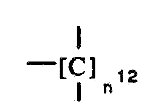 gebunden ist
W ein heterocyclischer Ring, ausgewählt aus: 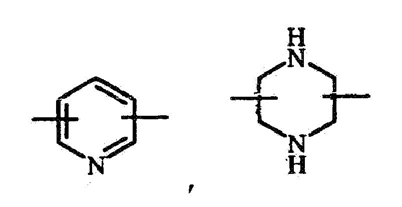 ist.

3. Verbindung der Formel (I) gemäß Anspruch 1, worin:
X folgendes ist:
a) gradkettiges oder verzweigtes C₁-C₁₀-Alkylen, ggf. subsituiert mit einer oder mehreren -ONO₂-Gruppen;
c) 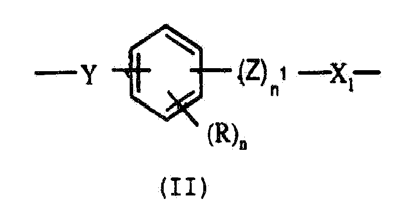 worin:
Y ein geradkettiges oder verzweigtes C₁-C₆-Alkylen oder -CH=CH-(CH₂)ₙ²- ist, worin n² 0 oder 1 ist;
R H oder -OR' ist, worin R' CH₃ ist;
Z O oder -OC(O)- ist;
n 0 oder 1 ist;
n¹ 0 oder 1 ist;
X₁ ein geradkettiges oder verzweigtes C₁-C₆-Alkylen ist, das ggf. mit einer oder mehreren -ONO₂-Gruppen substituiert ist, oder X₁ ist eine Gruppe der Formel (III) 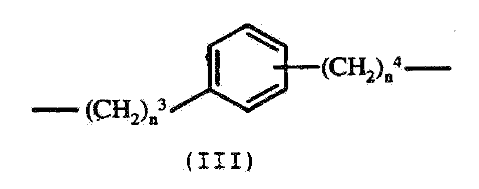
worin:
n³ 0 bis 1 ist;
n⁴ 1 ist;
worin die Gruppe D der Formel (I) an die X₁-Gruppe der Formel (II) und an die -(CH₂)ₙ⁴⁻-Gruppe der Formel (III) gebunden ist;
d) 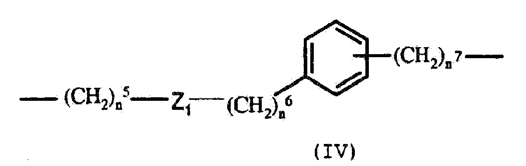
worin n⁵ eine ganze Zahl von 1 bis 5 ist;
Z₁ -C(O)O- oder -OC(O)- ist;
n⁶ eine ganze Zahl von 0 bis 5 ist;
n⁷ eine ganze Zahl von 1 bis 5 ist;
worin die Gruppe D der Formel (I) an die -(CH₂)ₙ⁷⁻-Gruppe gebunden ist;
e) 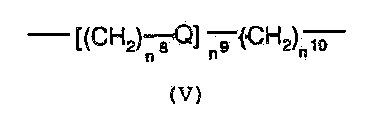 worin
Q O ist;
n⁸ 1 oder 2 ist;
n⁹ eine ganze Zahl von 1 bis 4 ist;
n¹⁰ eine ganze Zahl von 1 bis 2 ist.

4. Verbindung der Formel (I) gemäß Anspruch 1, ausgewählt aus der Gruppe:

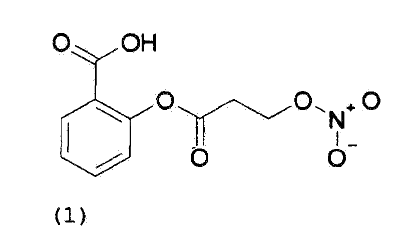

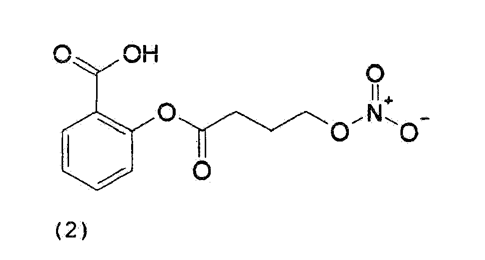

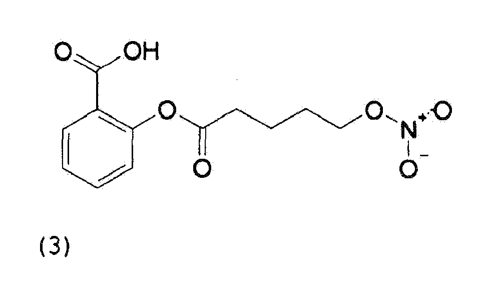

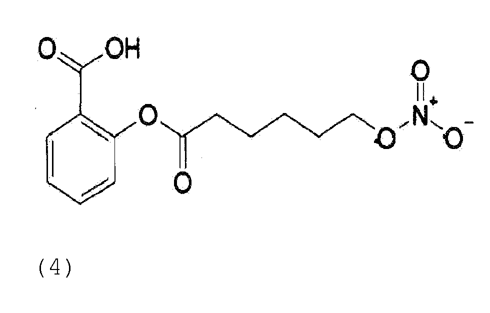

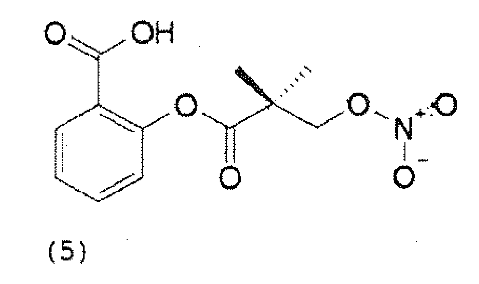

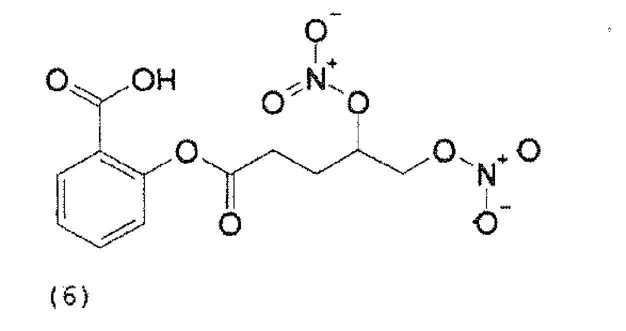

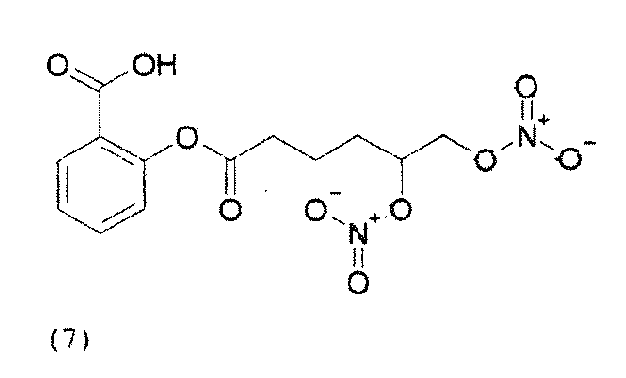

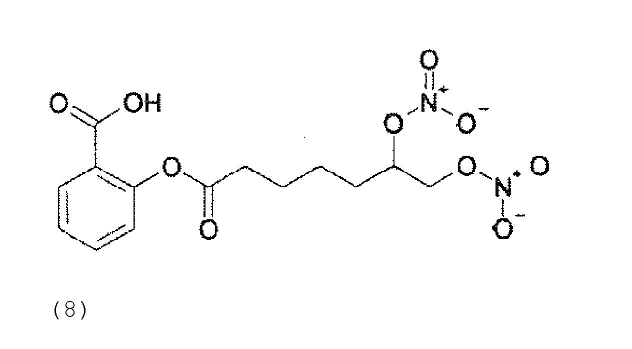

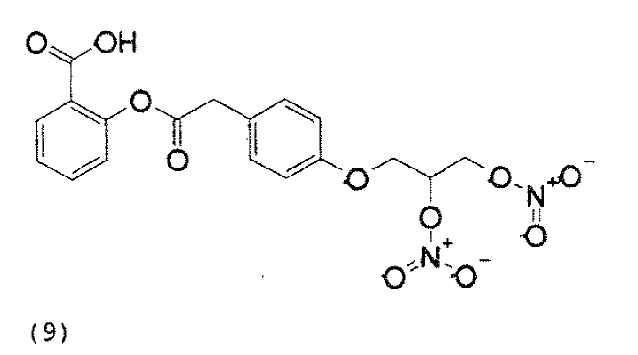

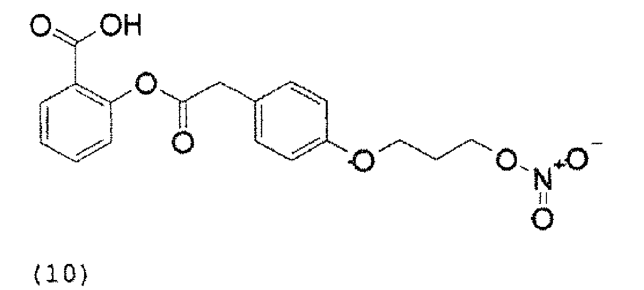

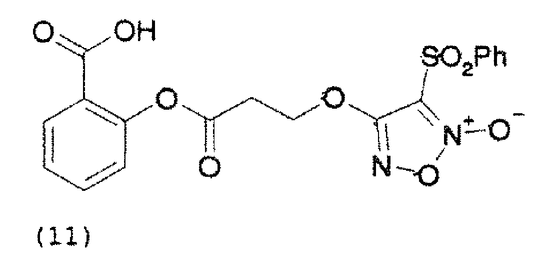

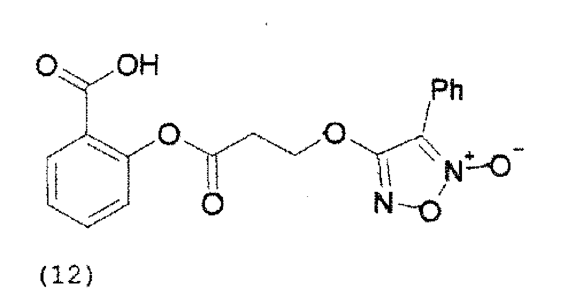

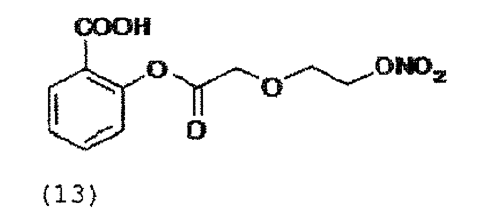

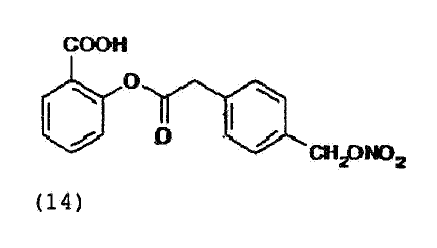

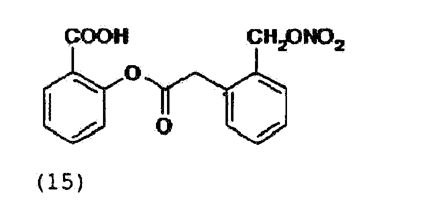

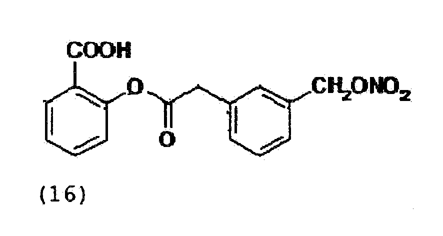

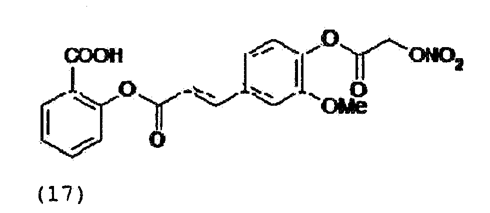

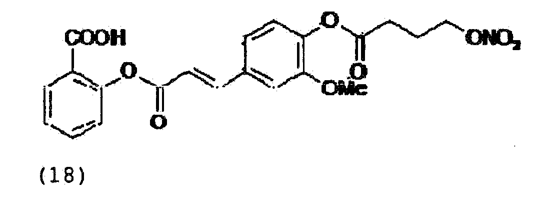

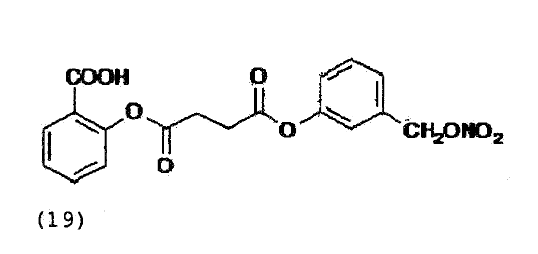

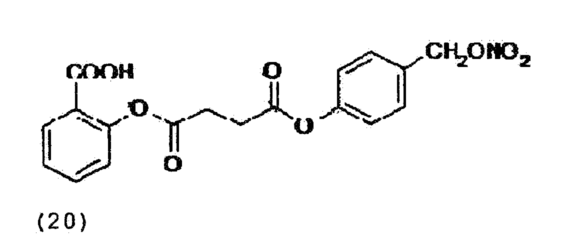

5. Verfahren zur Herstellung einer Verbindung der Formel (I) gemäß Anspruch 1, umfassend die Oxidation einer Verbindung der Formel (VIII): 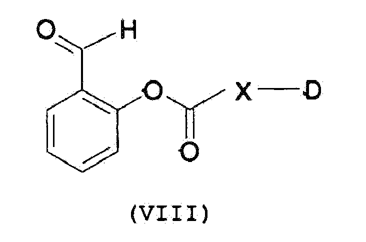 worin X und D wie in Anspruch 1 definiert sind.

6. Verbindung der allgemeinen Formel (I) gemäß den Ansprüchen 1 bis 4 zur Verwendung als Medikament.

7. Verwendung einer Verbindung gemäß den Ansprüchen 1 bis 4 zur Herstellung eines Medikaments mit entzündungshemmender, antithrombotischer und Thrombozytenaggregations-hemmender Aktivität.

8. Verwendung einer Verbindung gemäß den Ansprüchen 1 bis 4 zur Herstellung eines Medikaments zur Behandlung von Entzündungen, Schmerz, Fieber und kardiovaskulären Erkrankungen.

9. Verwendung einer Verbindung gemäß den Ansprüchen 1 bis 4 zur Herstellung eines Medikaments zur Vorbeugung oder Behandlung von Krebserkrankungen.

10. Verwendung einer Verbindung gemäß Anspruch 9 zur Herstellung eines Medikaments zur Behandlung von Darmkrebs, Blasenkrebs, Prostatakrebs.

11. Pharmazeutische Zusammensetzung, umfassend einen pharmazeutisch akzeptablen Träger und eine pharmazeutisch wirksame Menge einer wie in den Ansprüchen 1 bis 4 definierten Verbindung der allgemeinen Formel (I) und/oder eines Salzes oder Stereoisomers davon.

## Revendications

1. Composé de formule générale (I) et ses stéréoisomères ou sels pharmaceutiquement acceptables : 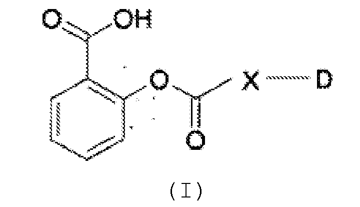 dans laquelle :
D est -ONO₂ ou 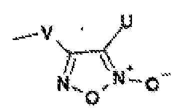 où V est -CH₂-, -O-, -S- ou -NH- ; U est un radical alkyle en C₁ à C₁₀ éventuellement substitué par -OH ou -NH₂, aryle, alcoxy en C₁ à C₁₀, aryloxy, thioalkyle en C₁ à C₁₀, thioaryle, halogéno, di (alkylamino en C₁ à C₁₀), diarylamino, aryl(alkylamino en C₁ à C₁₀), alkylsulfoxy en C₁ à C₁₀, arylsulfoxy, alkylsulfone en C₁ à C₁₀, arylsulfone, -CN, -NO₂, -NHCOR₀, -COR₀, -COOR₀, -CON(R₀) (R₁), où R₀ et R₁ sont identiques ou différents et sont H, un radical alkyle ou aryle ;
X est un radical divalent ayant les significations suivantes :
a) un radical alkylène en C₁ à C₂₀ linéaire ou ramifié, éventuellement substitué par un ou plusieurs substituants choisis dans l'ensemble constitué par les atomes d'halogène, -OH, -COOH, -ONO₂ et T, où T est -OC(O)-(alkyl en C₁ à C₁₀) -ONO₂ ou -O-(alkyl en C₁ à C₁₀)-ONO₂ ;
b) un groupe cycloalkylène en C₅ à C₇ éventuellement substitué par un groupe alkyle en C₁ à C₁₀ linéaire ou ramifié ;
c) 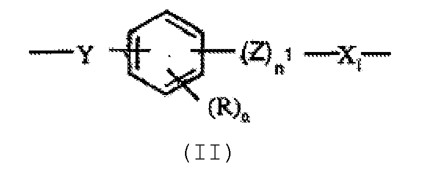 où :
Y est un radical alkylène en C₁ à C₂₀ linéaire ou ramifié, ou -CH=CH-(CH₂)ₙ²- où n² est un entier de 0 à 10 ;
R est H ou un radical alkyle en C₁ à C₅, -COOH ou -OR' où R' est H ou un groupe alkyle en C₁ à C₃ ;
Z est 0, -C(O)O- ou -OC(O)- ;
n vaut 0 ou 1 ;
n¹ vaut 0 ou 1 ;
X₁ est un radical alkylène en C₁ à C₂₀ linéaire ou ramifié, éventuellement substitué par un ou plusieurs substituants choisis dans l'ensemble constitué par les atomes d'halogène, -OH, -COOH, -ONO₂, ou bien X₁ est un groupe de formule (III) 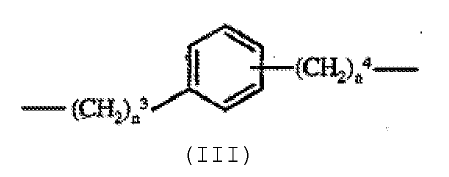
dans laquelle :
n³ est un entier de 0 à 5 ;
n⁴ est un entier de 1 à 5 ;
le groupe D dans la formule (I) étant lié au groupe X₁ de la formule (II), et au groupe - (CH₂)ₙ₄- de la formule (III) ;
d) 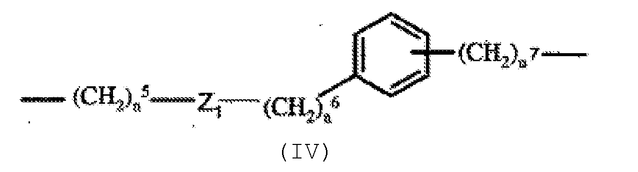
où n⁵ est un entier de 1 à 20 ;
Z₁ est -C(O)O- ou -OC(O)- ;
n⁶ est un entier de 0 à 20 ;
n⁷ est un entier de 1 à 20 ;
le groupe D dans la formule (I) étant lié au groupe -(CH₂)ₙ₇- ;
e) 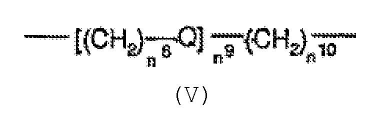 où :
Q est 0 ou S ;
n⁸ est un entier de 1 à 6 ;
n⁹ est un entier de 1 à 10 ;
n¹⁰ est un entier de 1 à 10 ;
f) 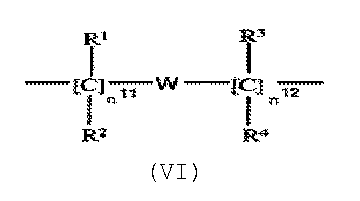 où :
n¹¹ est un entier de 0 à 10 ;
n¹² est un entier de 1 à 10 ; R¹, R², R³, R⁴ sont identiques ou différents et représentent H ou un radical alkyle en C₁ à C₄ linéaire ou ramifié ;
le groupe D de la formule (I) étant lié à : 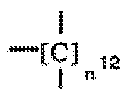
W est un cycle à 5 ou 6 chaînons hétérocyclique saturé, insaturé ou aromatique, contenant un ou plusieurs hétéroatomes choisis parmi l'azote, l'oxygène, le soufre, et est choisi parmi :

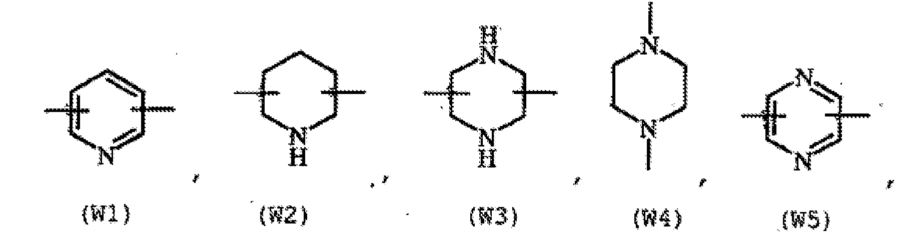

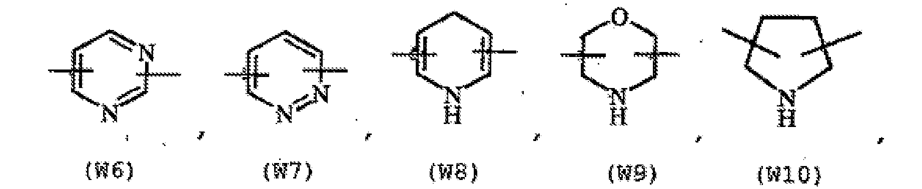

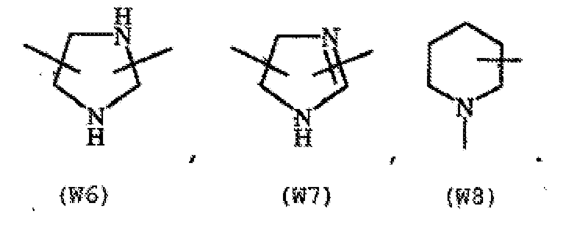

2. Composé de formule (I) selon la revendication 1, dans lequel :
X est :
a) un radical alkylène en C₁ à C₁₀ linéaire ou ramifié, éventuellement substitué par un ou plusieurs substituants choisis dans l'ensemble constitué par les atomes d'halogène, -OH, -COOH, -ONO₂ et T, où T est -OC(O)-(alkyl en C₁ à C₁₀)-ONO₂ ou -O-(alkyl en C₁ à C₁₀)-ONO₂ ;
c) 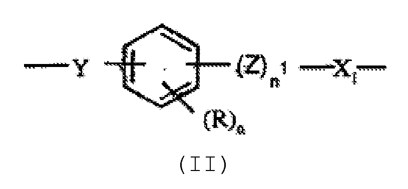 où :
Y est un radical alkylène en C₁ à C₆ linéaire ou ramifié, ou -CH=CH-(CH₂)ₙ₂- où n² vaut 0 ou 1 ;
R est H, -CH₃, -COOH ou -OR' où R' est H ou -CH₃ ;
Z est 0 ou -OC(O)- ;
n vaut 0 ou 1 ;
n¹ vaut 0 ou 1 ;
X₁ est un radical alkylène en C₁ à C₁₀ linéaire ou ramifié, éventuellement substitué par un ou plusieurs substituants choisis dans l'ensemble constitué par les atomes d'halogène, -OH, -COOH, -ONO₂, ou bien X₁ est un groupe de formule (III) : 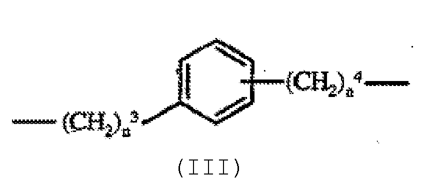
dans laquelle :
n³ vaut 0 ou 1 ;
n⁴ vaut 1 ;
le groupe D dans la formule (I) étant lié au groupe X₁ de la formule (II), et au groupe - (CH₂)ₙ₄- de la formule (III) ;
d) 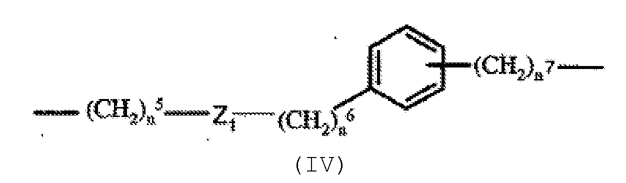
où n⁵ est un entier de 1 à 10 ;
Z₁ est -C(O)O- ou -OC(O)- ;
n⁶ est un entier de 0 à 10 ;
n⁷ est un entier de 1 à 10 ;
le groupe D dans la formule (I) étant lié au groupe - (CH₂)ₙ₇- ;
e) 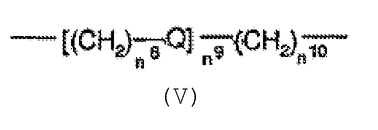 où :
Q est -O- ou -S- ;
n⁸ est un entier de 1 à 4 ;
n⁹ est un entier de 1 à 6 ;
n¹⁰ est un entier de 1 à 6 ;
f) 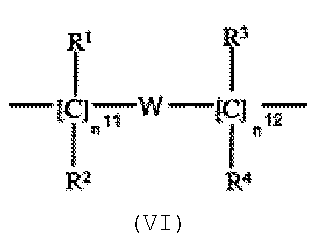 où :
n¹¹ est un entier de 0 à 4 ;
n¹² est un entier de 1 à 4 ;
R¹, R², R³, R⁴ sont H ;
le groupe D de la formule (I) étant lié à : 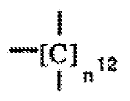 W est un hétérocycle choisi parmi :

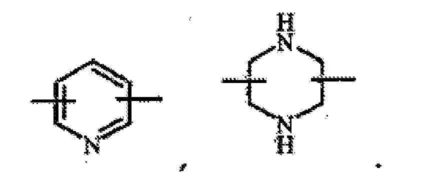

3. Composé de formule (I) selon la revendication 1, dans lequel :
X est :
a) un radical alkylène en C₁ à C₁₀ linéaire ou ramifié, éventuellement substitué par un ou plusieurs groupes -ONO₂ ;
c) 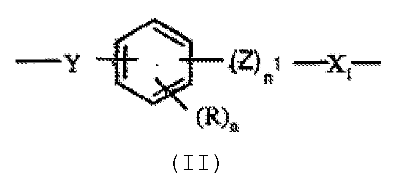 où :
Y est un radical alkylène en C₁ à C₆ linéaire ou ramifié, ou -CH=CH-(CH₂)ₙ₂- où n² vaut 0 ou 1 ;
R est H ou -OR', où R' est CH₃ ;
Z est 0 ou -OC(O)- ;
n vaut 0 ou 1 ;
n¹ vaut 0 ou 1 ;
X₁ est un radical alkylène en C₁ à C₆ linéaire ou ramifié,
éventuellement substitué par un ou plusieurs groupes -ONO₂, ou bien X₁ est un groupe de formule (III) : 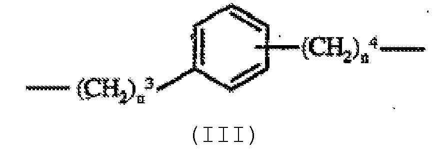
dans laquelle :
n³ vaut 0 ou 1 ;
n⁴ vaut 1 ;
le groupe D dans la formule (I) étant lié au groupe X₁ de la formule (II), et au groupe - (CH₂)ₙ₄- de la formule (III) ;
d) 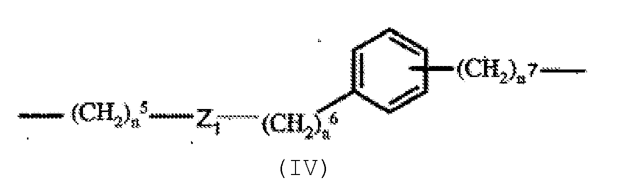 où n⁵ est un entier de 1 à 5 ;
Z₁ est -C(O)O- ou -OC(O)- ;
n⁶ est un entier de 0 à 5 ;
n⁷ est un entier de 1 à 5 ;
le groupe D dans la formule (I) étant lié au groupe -(CH₂)ₙ₇- ;
e) 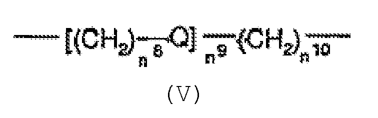 où :
Q est 0 ;
n⁸ vaut 1 ou 2 ;
n⁹ est un entier de 1 à 4 ;
n¹⁰ est un entier de 1 à 2.

4. Composé de formule (I) selon la revendication 1, choisi parmi les suivants :

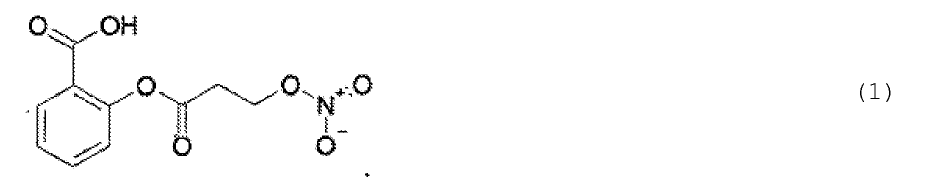

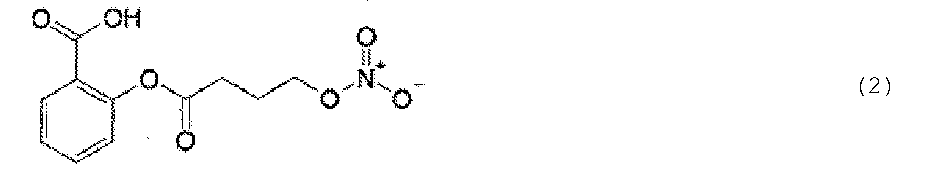

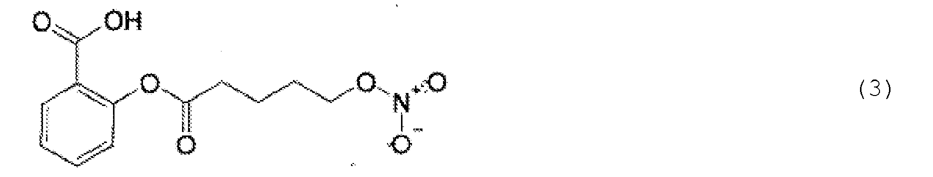

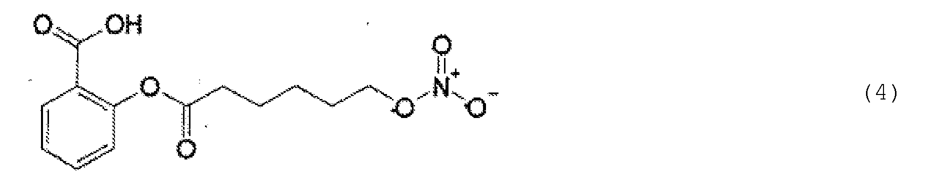

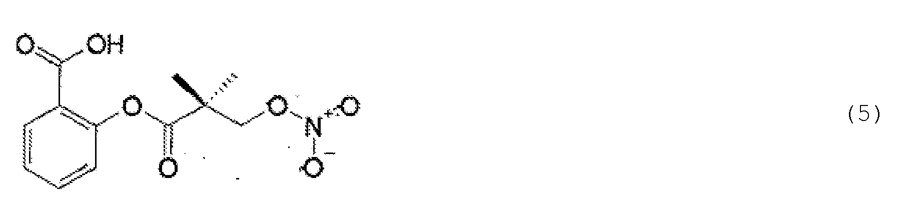

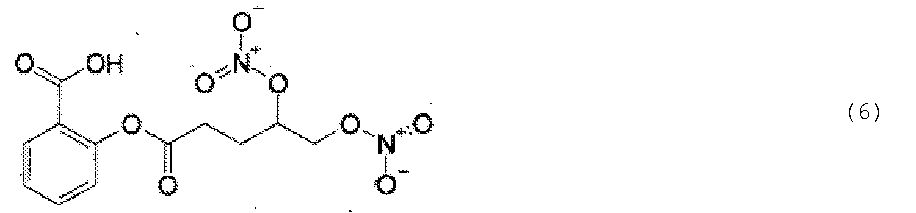

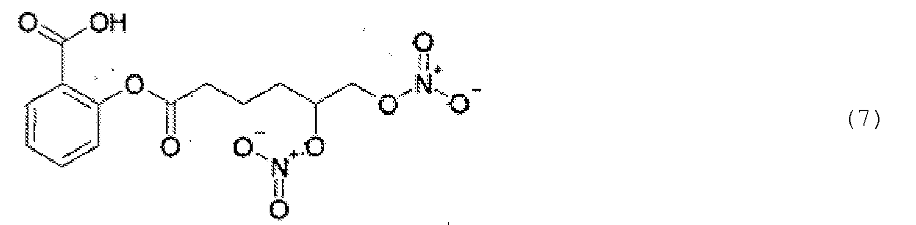

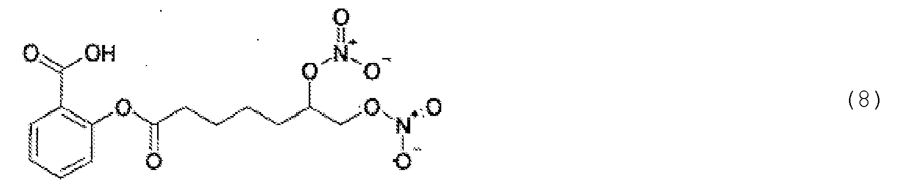

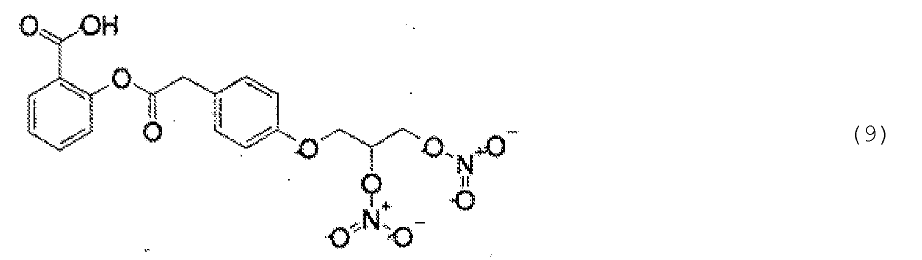

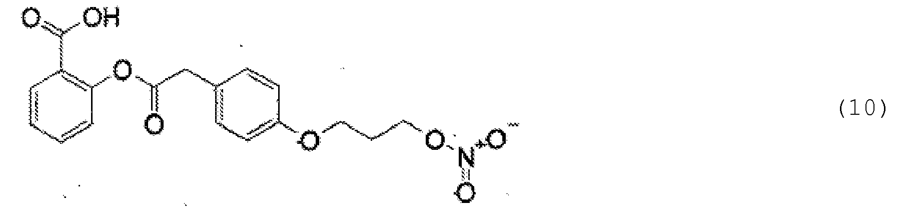

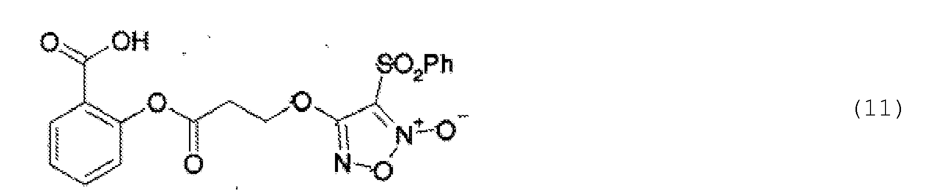

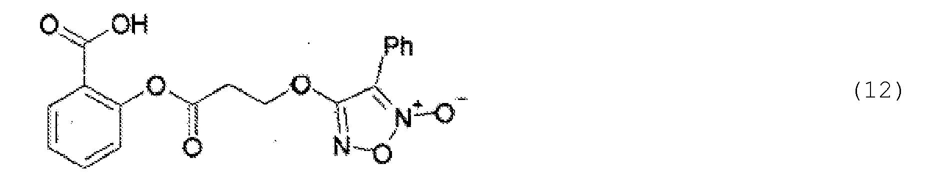

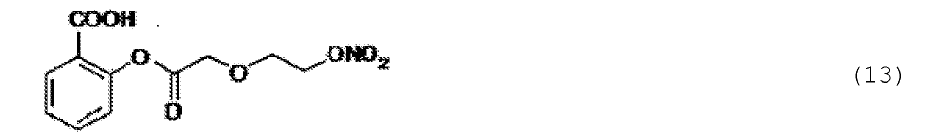

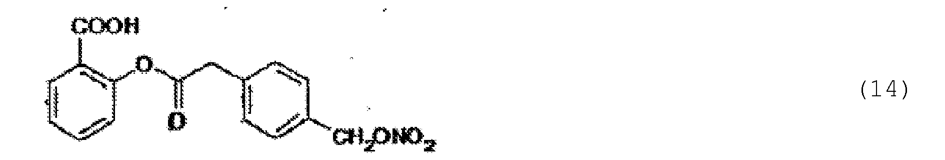

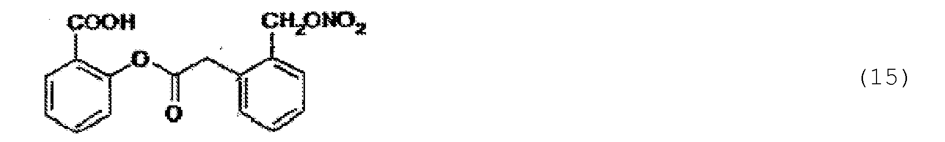

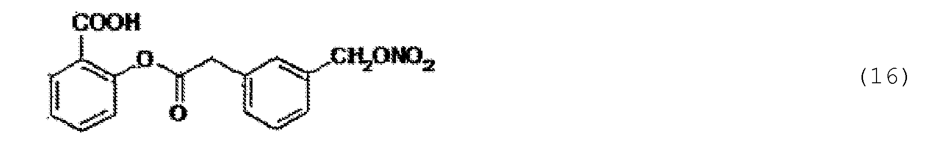

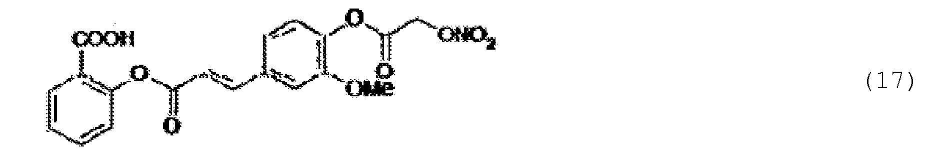

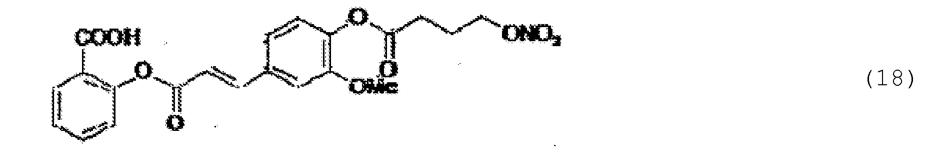

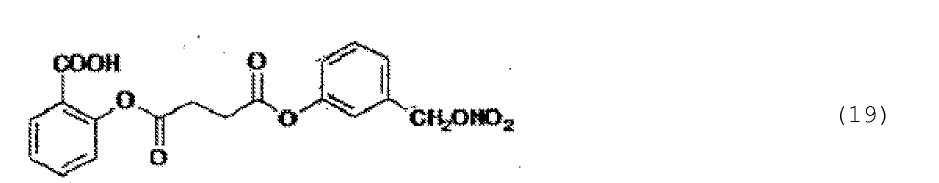

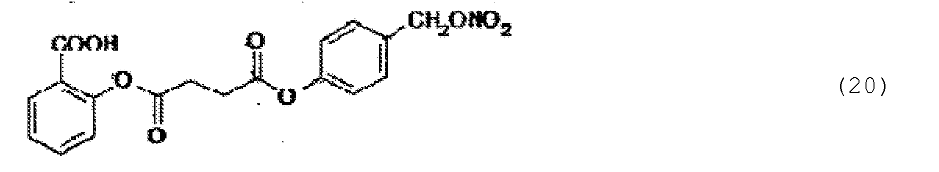

5. Procédé pour préparer un composé de formule (I) selon la revendication 1, comprenant l'oxydation d'un composé de formule (VIII) _{:} 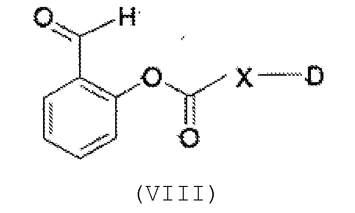 dans laquelle X et D sont tels que définis dans la revendication 1.

6. Composé de formule générale (I) selon les revendications 1 à 4, pour utilisation en tant que médicament.

7. Utilisation d'un composé selon les revendications 1 à 4 pour la préparation d'un médicament ayant une activité anti-inflammatoire, antithrombotique et antiplaquettaire.

8. Utilisation d'un composé selon les revendications 1 à 4 pour la préparation d'un médicament destiné au traitement d'une inflammation, d'une douleur, d'une fièvre et de maladies cardiovasculaires.

9. Utilisation d'un composé selon les revendications 1 à 4 pour la préparation d'un médicament destiné à prévenir ou traiter des maladies cancéreuses.

10. Utilisation d'un composé selon la revendication 9 pour la préparation d'un médicament destiné au traitement du cancer du côlon, du cancer de la vessie, du cancer de la prostate.

11. Composition pharmaceutique comprenant un véhicule pharmaceutiquement acceptable et une quantité pharmaceutiquement efficace d'un composé de formule générale (I) et/ou d'un stéréoisomère ou sel de celui-ci, tel que défini dans les revendications 1 à 4.
